# FASCICULE DE BREVET EUROPEEN

(11) **EP 1 387 841 B1**
(45) Date de publication et mention de la délivrance du brevet: **08.03.2006**
(21) Numéro de dépôt: 02730384.1
(22) Date de dépôt: 02.05.2002
(51) Int. Cl.: C07D 405/06, C07D 405/14, C07D 409/14, C07F 9/6558, A61K 31/4725, A61K 31/4164, A61P 31/10

(54) **NOUVEAUX DERIVES D'AZOLE OU DE TRIAZOLE, LEUR PROCEDE DE PREPARATION ET LEUR APPLICATION COMME FONGICIDES**
AZOL- ODER TRIAZOL-DERIVATE, VERFAHREN ZU DEREN HERSTELLUNG UND IHRE VERWENDUNG ALS FUNGIZIDE
NOVEL AZOLE OR TRIAZOLE DERIVATIVES, METHOD FOR PREPARING THE SAME AND USE THEREOF AS FUNGICIDES

(30) Priorité: 04.05.2001 FR 0105958
(43) Date de publication de la demande: 11.02.2004
(73) Titulaire: Aventis Pharma S.A., 92160 Antony (FR)
(72) Inventeur: BABIN, Didier, F-78180 Montigny (FR); WESTON, John, D-65779 Kelkheim (DE)
(74) Mandataire: Rousseau, Pierrick Edouard
(86) Numéro de dépôt international: PCT/FR2002/001521
(87) Numéro de publication internationale: WO 2002/090354

(56) Documents cités:
- EP-A- 0 050 298
- EP-A- 0 121 753
- WO-A-00/20413
- WO-A-01/74808

## Description

La présente invention concerne de nouveaux dérivés d'azole ou de triazole, leur procédé de préparation et leur application comme fongicides.

De nombreux composés ayant une activité antifongique sont connus dans l'art antérieur. On peut citer notamment des dérivés d'azoles tels que définis dans les demandes suivantes : EP 0 121 753 A (Hoechst AG), EP 0 050 298 A (Hoechst AG), US 2,813,872 (J schmutz), WO 00/20413 (Hoechst Marion Roussel). Par ailleurs, les nouveaux composés antifongiques doivent pouvoir présenter une solubilité améliorée et doivent pouvoir également être plus facilement absorbés. Il existe néanmoins un réel besoin de mettre en oeuvre de nouveaux composés antifongiques, les souches actuelles pouvant être ou devenir résistantes aux agents conventionnels notamment lorsque ces derniers ne possèdent qu'une activité fongistatique. Enfin, l'incidence du *Candida albicans* comme agent infectieux, est de plus en plus importante, notamment vis-à-vis des patients immunodéprimés, par exemple suite à une infection au VIH, et nécessite donc de nouveaux traitements.

L'objectif de la présente invention est de fournir de nouveaux composés ayant une activité antifongique, notamment vis-à-vis des souches *Candida* ou *Aspergillus.*

L'invention a pour objet, les composés de formule (I) 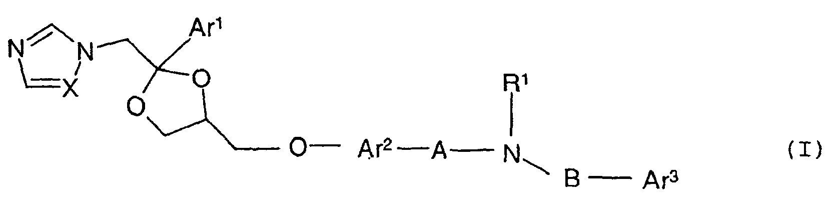
dans laquelle
- X est un atome d'azote ou un groupement CH,
- Ar¹ représente un aryle carbocyclique ou hétérocyclique non substitué ou substitué par un ou plusieurs radicaux R², R³
ou R⁴
- Ar² représente un phénylène ou naphtylène non substitué ou substitué par un ou plusieurs radicaux R⁵, R⁶ ou R⁷
- Ar³ représente un aryle carbocyclique ou hétérocyclique non substitué ou substitué par un ou plusieurs radicaux R⁸, R⁹
ou R¹⁰
- A représente un radical (C₁-C₄)-alkylène ou un radical C(O),
- B représente un radical (C₁-C₉)-alkylène-CH=CH- ou un radical (C₁-C₄)-alkylène-cyclopropylène, les dits radicaux cyclopropylène ou -CH=CH- étant non substitués ou substitués par un radical R² et/ou R³,
- R¹ représente un atome d'hydrogène, un groupement -SO₃H ou bien un radical (C₁-C₆)-alkyle non substitué ou substitué par un radical tel que défini pour R²
- R², R³, R⁴, R⁵, R⁶, R⁷, R⁸, R⁹ ou R¹⁰ identiques ou différents, représentent fluor, chlore, brome, cyano, mono- bi- ou trihalogeno(C₁-C₈)alkyle, mono- bi- ou trihalogeno(C₁-C₈)-alkyloxy, hydroxy, nitro, carboxyle, formyle, -SO₃H, -OSO₃H, (R¹¹O)₂P(O)-, (R¹¹O)₂P(O)-O-, amino, (C₁-C₈)-alkylamino, di((C₁-C₈)alkyl)amino, (C₅-C₁₄)-aryl-(C₁-C₆)-alkylènamino ou (C₅-C₁₄)-arylamino, (C₁-C₈)-alkyle, (C₅-C₁₄)-aryle, un hétérocycle éventuellement substitué par oxo, (C₅-C₁₄)-aryl- (C₁-C₆) alkyle, amino-(C₁-C₆) -alkyle, (C₁-C₈)-alkylamino- (C₁-C₆) -alkyle, di-((C₁-C₈)alkyl)amino-(C₁-C₆)-alkyle, hydroxy-(C₁-C₆) alkyle, (C₁-C₆)-alkyloxy- (C₁-C₆) - alkyle, (C₁-C₈)-alkyloxy éventuellement interrompu par un ou plusieurs atomes d'oxygène, (C₅-C₁₄)-aryl-(C₁-C₆)-alkylènoxy, (C₅-C₁₄)-aryloxy, hydroxy-(C₁-C₆)alkylènoxy, (C₁-C₆) -alkyloxy- (C₁-C₆) alkylènoxy, amino-(C₁-C₆) -alkylènoxy, (C₁-C₆ )-alkylamino-(C₁-C₆)-alkylènoxy, di((C₁-C₈)-alkyl)amino-(C₁-C₆)-alkylènoxy, méthylènedioxy, (C₁-C₆)-alkyloxycarbonyle, (C₁-C₆)-alkylcarbonyle, (C₅-C₁₄) aryl-(C₁-C₆)-alkylènecarbonyl, (C₅-C₁₄) -aryl-carbonyle, (C₁-C₆)-alkylaminocarbonyle, (C₁-C₆)alkanoylamino, (C₁-C₆)-alkylsulfonylamino, (C₅-C₁₄)-arylsulfonylamino, (C₅-C₁₄)-aryl-(C₁-C₆)-alkylènesulfonylamino, (C₁-C₆)-alkylaminosulfonyle, (C₅-C₁₄)-aryl-(C₁-C₅)-alkylènaminosulfonyl, (C₁-C₆)-alkylsulfonyl, (C₅-C₁₄)-aryl-(C₁-C₈)-alkylènesulfonyle ou (C₅-C₁₄)-aryl-sulfonyle, les dits radicaux alkyles, aryles ou hétérocycles étant eux même non substitués ou substitués par un ou plusieurs groupements cités ci-dessus.
- R¹¹ représente hydrogène, (C₁-C₁₀)-alkyl, (C₆-C₁₄) -aryle ou (C₆-C₁₄)-aryl-(C₁-C₆)-alkyle,
sous toutes leurs formes stéréoisomères possibles et leurs mélanges,ainsi que leurs sels d'addition physiologiquement acceptables et leurs prodrogues.

Tous les radicaux qui peuvent se retrouver plusieurs fois dans les composés de formule (I), par exemple, le radical R², sont indépendants les uns des autres et peuvent être identiques ou différents.

Les radicaux alkyles cités plus haut peuvent être linéaires ou ramifiés. Ceci s'applique également lorsqu'ils portent un substituant ou lorsqu'ils sont inclus dans des groupements tels que par exemple alkoxy, alkoxycarbonyle ou aralkyle.

Par (C₁-C₈)-alkyle on entend les radicaux méthyle, éthyle, propyle, butyle, pentyle, hexyle, heptyle, octyle, nonyle, les n-isomères de ces radicaux, isopropyle, isobutyle, isopentyle, néopentyle, isohexyle, 3-méthylepentyle, 2,3,4-triméthylhexyle, sec-butyle, tert-butyle, tert-pentyle. Parmi les radicaux préférés on peut citer méthyle, éthyle, n-propyle, isopropyle, n-butyle, isobutyle, et tert-butyle. Par (C₁-C₆)-alkyle on entend les radicaux méthyle, éthyle, propyle, butyle, pentyle, hexyle et les n-isomères de ces radicaux.

Par radical alkyloxy interrompu par un ou plusieurs atomes d'oxygènes, on entend de préférence des radicaux du type O-CH₂-O-(CH₂)₂-O-CH₃.

Les radicaux alkylènes bivalents correspondant aux radicaux monovalents cités plus haut sont par exemple les radicaux méthylène, éthylène, 1,3-propylène, 1,2-propylène (=₁-méthyléthylène), 2,3-butylène (=1,2-diméthyléthylène), 1,4-butylène, ou 1,6-hexylène.

Par radicaux alkylène bivalents insaturés on entend les radicaux alkénylène et alkynylène qui peuvent également être linéaires ou ramifiés. Il s'agit par exemple des radicaux vinylène, propénylène, éthynylène ou propynylène.

Sauf indication contraire, les radicaux alkyles ou cycloalkyles peuvent être non substitués ou substitués par un ou plusieurs radicaux identiques ou différents choisis parmi (C₁-C₆)-alkyle, (C₁-C₆)-alkoxy, hydroxyle, halogène tel que fluor, chlore et brome, nitro, amino, trifluorométhyle, mono, -OCF₃, cyano, carboxyl, -SO₃H, -OSO₃H, PO₃H₂, OPO₃H₂, (C₁-C₄)-alkoxycarbonyle, phényle, phénoxy, benzyle et benzyloxy. Bien entendu ceci s'applique également lorsque le radical alkyle fait parti d'un groupement le renfermant, par exemple tel que, (C₁-C₆)-alkyloxycarbonyle, (C₁-C₆)-alkylcarbonyle ou (C₁-C₆)-alkylaminocarbonyle

Par halogène on entend fluor, chlore, brome ou iode.

Par le terme aryle on entend :
- soit les radicaux (C₅-C₁₄)-aryle hétérocycliques (= (C₅-C₁₄)-hétéroaryle), dans lesquels les atomes de carbone du cycle sont remplacés par un hétéroatome tel que azote, oxygène ou soufre,
- soit les radicaux (C₆-C₁₄)-aryle carbocyclique.

Parmi les radicaux(C₆-C₁₄)-aryle carbocycliques, on peut citer le phényle, le naphtyle, le biphénylyle, l'anthryle ou le fluorényle et tout particulièrement le 1-naphtyle, le 2-naphtyle et le phényle.

Sauf indication contraire, les radicaux aryles, en particulier phényle, peuvent être non substitués ou substitués par un ou plusieurs radicaux identiques ou différents choisis parmi (C₁-C₆)-alkyle, (C₁-C₆)-alkoxy, hydroxyle, hydroxy(C₁-C₆)-alkyle, halogène tel que fluor, chlore et brome, nitro, amino, trifluorométhyle, -OCF₃, cyano, carboxyl, -SO₃H, -OSO₃H, PO₃H₂, OPO₃H₂, (C₁-C₄)-alkoxycarbonyle, phényle, phénoxy, benzyle, benzyloxy et méthylènedioxy.

Dans le cas du phényle monosubstitué, le substituant peut être situé en position 2, 3 ou 4, et de préférence en position 3 ou 4. De préférence, Ar³ représente un phériyle substitué en position 4. Dans le cas où le phényle est di-

Parmi les radicaux(C₆-C₁₄)-aryle carbocycliques, on peut citer le phényle, le naphtyle, le biphénylyle, l'anthryle ou le fluorényle et tout particulièrement le 1-naphtyle, le 2-naphtyle et le phényle.

Sauf indication contraire, les radicaux aryles, en particulier phényle, peuvent être non substitués ou substitués par un ou plusieurs radicaux identiques ou différents choisis parmi (C₁-C₆)-alkyle, (C₁-C₆)-alkoxy, hydroxyle, hydroxy(C₁-C₆)-alkyle, halogène tel que fluor, chlore et brome, nitro, amino, trifluorométhyle, -OCF₃, cyano, carboxyl, -SO₃H, -OSO₃H, PO₃H₂, OPO₃H₂, (C₁-C₄)-alkoxycarbonyle, phényle, phénoxy, benzyle, benzyloxy et méthylènedioxy.

Dans le cas du phényle monosubstitué, le substituant peut être situé en position 2, 3 ou 4, et de préférence en position 3 ou 4. De préférence, Ar³ représente un phényle substitué en position 4. Dans le cas où le phényle est disubstitué, les substituants peuvent être en position 2,3 ou 2,4 ou 2,5 ou 2,6 ou 3,4 ou 3,5. De préférence, lorsque Ar¹ représente un phényle disubstitué, les deux substituants sont en position 2,4. Lorsque ce phényle est tri-substitué les positions sont les suivants : 2,3,4 ou 2,3,5 ou 2,3,6 ou 2,4,5 ou 2,4,6 ou 3,4,5. De la même manière, les radicaux naphtyles ou d'autres radicaux aryles peuvent être substitués en toute position, par exemple le radical 1-naphtyle en position 2-, 3-, 4-, 5-, 6-, 7-, et 8 et le radical 2-naphtyle en position 1-, 3-, 4-, 5-, 6-, et 7.

Le groupement (C₅-C₁₄)-aryle peut représenter également un système aromatique monocyclique ou polycyclique dans lequel 1,2,3 ou 4 atomes de carbone du cycle sont remplacés par des hétéroatomes, en particulier identiques ou différents du groupe constitué de l'azote, l'oxygène et le soufre. Parmi les groupements (C₅-C₁₄)-aryle hétérocycliques (= (C₅-C₁₄)-hétéroaryle) on peut citer les groupements 2-pyridyle, 3-pyridyle, 4-pyridyle, pyrrolyle, furyle, thiényle, imidazolyle, pyrazolyle, oxazolyle, isoxazolyle, thiazolyle, isothiazolyle, tétrazolyle, pyridyle, pyrazinyle, pyrimidinyle, indolyle, isoindolyle, indazolyle, phtalazinyle, quinolyle, isoquinolyle, quinoxalinyle, quinazolinyle, cinnolinyle, β-carbolinyle, ou encore des dérivés benzo-condensés, cyclopenta-, cyclohexa-, ou cyclohepta- condensés de ces radicaux.

Le système hétérocyclique peut être substitué par les mêmes substituants cités plus haut pour le système carbocyclique.
Bien entendu, le description ci-dessus concernant les groupes aryles s'applique également lorsque aryle est un radical inclus dans un groupement tel que aryl-alkyle. Comme exemples préférés de groupements aryl-alkyle, on peut citer benzyle, 1-phényléthyl ou 2-phényléthyle.

Par hétérocycle, on entend de préférence un radical à 5 ou 6 chaînons, non aromatique, renfermant éventuellement une ou deux doubles liaisons et un ou plusieurs atomes d'azote ou d'oxygène substitué ou non substitué par les mêmes substituants cités plus haut pour le système carbocyclique ainsi que le radical oxo. L'invention comprend ainsi les hétérocycles suivants :

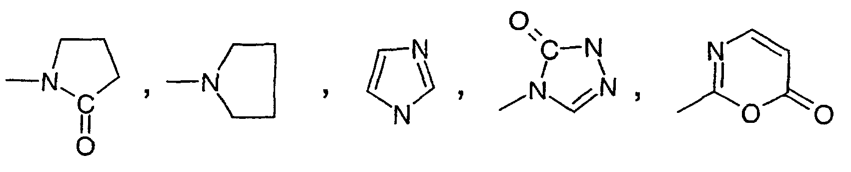

Ces hétérocycles pouvant être substitués. Il peut alors s'agir des radicaux suivants : 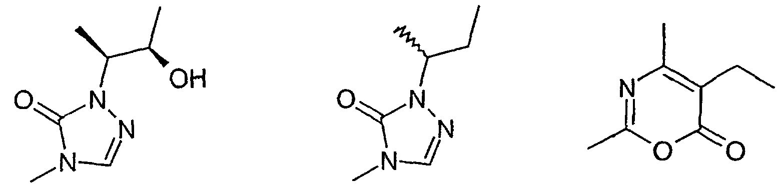 Les atomes de carbone optiquement actifs contenus dans les composés de formule (I) peuvent indépendamment les uns des autres présenter la configuration R ou la configuration S.

Les composés de formule (I) peuvent être sous la forme d'énantiomères purs ou de diastéréoisomères purs ou sous la forme d'un mélange d'énantiomères, par-exemple sous forme de racémates ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I) et tous les rapports de ces stéréoisomères au sein des dits mélanges.

Les composés de formule (I) peuvent le cas échéant être présent sous forme d'isomères E ou d'isomères Z. L'invention a donc pour objet les isomères E purs, les isomères Z purs et les mélanges E/Z selon un rapport quelconque. Lorsque les composés de formule (I) renferment un cyclopropane, ces composés de formule (I) peuvent être présent sous forme d'isomères cis ou trans. L'invention a donc pour objet les isomères cis purs, les isomères trans purs et les mélanges cis/trans selon un rapport quelconque.

L'invention comprend également toutes les formes tautomères des composés de formule (I).Les diastéréoisomères, incluant les isomères E/Z (ou cis/trans) peuvent être séparés en isomères individuels, par exemple par chromatographie. Les racémates peuvent être séparés en deux énantiomères par des méthodes courantes telles que la chromatographie en phase chirale ou par des méthodes de résolution.

Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que l'acide carboxylique, il s'agit par exemple des sels de métaux alcalins ou alcalino terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyle)amine.

Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléique, fumarique, tartarique, méthanesulfonique ou para toluène sulfonique.

Les composés de formule (I) qui comportent un groupe basique et un groupe acide, tel que par exemple guanidino et carboxylique, peuvent être présents sous forme de Zwiterions (bétaînes), qui sont également inclus dans la présente invention.

Lorsque les composés de formule (I) renferment un groupement ammonium chargé, le contre anion (Q⁻) est de préférence un anion monovalent ou un équivalent d'anion polyvalent d'un acide organique ou inorganique non toxique, physiologiquement acceptable et en particulier pharmaceutiquement acceptable, par exemple l'anion ou un équivalent d'anion d'un des acides cités plus haut utile pour la formation des sels d'addition. Q⁻ peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et para-toluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant ou un dispersant ou à partir d'un autre sel par échange de cation ou d'anion.

L'invention inclut également tous les sels des composés de formule (I) qui, à cause de leur faible acceptabilité physiologique, ne sont pas directement utilisable comme médicament, mais sont utilisables comme produits intermédiaires pour mettre en oeuvre des modifications chimiques ultérieures au niveau des composés de formule (I) ou comme produits de départ pour la préparation de sels physiologiquement acceptables.

La présente invention inclut également tous les solvates des composés de formule (I) par exemples les hydrates, les solvates formés avec les alcools, et tous les dérivés des composés de formule (I), par exemple les esters, prodrogues et autres dérivés physiologiquement acceptables, ainsi que les métabolites des composés de formule (I).

L'invention a également pour objet les prodrogues des composés de formule (I) qui peuvent être transformées en composés de formule (I) in vivo dans les conditions physiologiques. Les prodrogues des composés de formule (I), à savoir les dérivés chimiquement modifiés des composés de formule (I) afin d'obtenir des propriétés améliorées de manière désirée, sont connus de l'homme du métier.

Pour avoir plus d'information sur le type de prodrug envisagé dans la présente invention, on peut citer les ouvrages suivants : Fleicher et al., Advanced Drug Delivery Review 19 (1996) 115-130; Design of prodrugs, H. Bundgaard, Ed., Elsevier, 1985; H. Bungaard, Drugs of the Future 16 (1991) 443; Saulnier et al. Bioorg. Med. Chem. Lett. 4 (1994) 1985; Safadi et al. Pharmaceutical Res. 10 (1993) 1350. Parmi les prodrugs appropriées des composés de formule (I) on peut citer de préférence :
- les prodrogues sous forme d'esters des groupes carboxyliques, sulfonique ou phosphonique, lorsque, par exemple, Ar³ est substitué respectivement par un groupement
- CO₂H, -SO₃H ou -PO₃H.
- les prodrogues sous forme d'acyle et de carbamate pour les groupes contenant un azote acylable tel que les groupes amino ou guanidine.
- les prodrogues sous forme de dérivés quaternaires d'azote cyclique ou non (benzyl substitué).
Dans les prodrogues acylés ou sous forme de carbamate, une ou plusieurs fois, par exemple deux fois, un atome d'hydrogène situé sur l'atome d'azote est remplacé par un groupe acyle ou carbamate. Parmi les groupes acyles ou carbamates préférés, on peut citer les groupes R₁₂CO-, R₁₃OCO-, dans lesquels R₁₂ est un hydrogène ou un radical (C₁-C₁₈)-alkyle, (C₃-C₁₄)-cycloalkyle, (C₃-C₁₄)-cycloalkyle-(C₁-C₈)-alkyle, (C₅-C₁₄)-aryle, dans lequel 1 à 5 atomes de carbone peuvent être remplacés par des hétéroatomes tels que N, O, S ou (C₅-C₁₄)-aryle-(C₁-C₈)alkyle, dans lequel 1 à 5 atomes de carbone dans la partie aryle peuvent être remplacés par des hétéroatomes tels que N, O, S et R₁₃ à les mêmes valeurs que R₁₂ à l'exception d'hydrogène.

Bien entendu, Ar¹ , Ar² , Ar³, R¹, R², R³, R⁴, R⁵, R⁶, R⁷, R⁸, R⁹, R¹⁰, R¹¹, R¹² et R¹³ peuvent adopter les définitions ci-dessus de manière indépendantes les unes par rapport aux autres.

Parmi les définitions concernant la formule (I), on peut citer les valeurs préférées suivantes :
Ar¹ et Ar³ représentent des groupements phényles,
Ar¹ représente un groupement phényle et Ar³ représente un hétérocycle
A est de préférence un groupement méthylène,
B est de préférence un groupement -CH₂-CH=CH- ou -CH₂₋(cyclopropyle)-, les dits groupements étant non substitués ou substitués par un ou plusieurs halogènes ou (C₁-C₄)-alkyle,
R¹ est de préférence un atome d'hydrogène ou un groupement méthyle ou éthyle non substitué ou substitué par fluor, -OH, -NH₂, (C₁-C₈) -alkyloxy, (C₁-C₈)-alkylamino, ou di-(C₁-C₈)-alkylamino, pyrrolidino ou 2-oxo-pyrrolidino.
R² et R³ sont de préférence des atomes d'halogène
R⁴ est de préférence un atome d'hydrogène
R⁶ est de préférence un atome d'hydrogène
R⁵ et R⁷ représentent de préférence hydrogène
R⁸, R⁹ et R¹⁰ représentent de préférence hydrogène, CN, halogène, -CF₃, -OCF₃, OH, -SO₃H, -P(O) (OH)₂, Carboxy, -OSO₃H, -OPO₃H, -NH₂, (C₁-C₆)-alkyle, un radical hétérocyclique saturé ou insaturé non aromatique, amino-(C₁-C₆)-alkyle, hydroxy-(C₁-C₆)-alkyle, (C₁-C₆)-alkyloxy, (C₁-C₆)-alkylamino-(C₁-C₆)-alkyloxy, (C₁-C₆)-alkyloxycarbonyle, (C₁-C₆)-alkylcarbonyle, (C₁-C₆)-alkylaminocarbonyle, (C₁-C₆)-alkylamino, di-(C₁-C₆)-alkylamino ou di-(C₁-C₆)-alkylamino-(C₁-C₆)-alkyloxy, les dits radicaux alkyles ou hétérocycles étant non substitués ou substitués par halogène, OH, SO₃H, P(O)(OH)₂, oxo, Carboxy, -OSO₃H, -OPO₃H₂, -NH₂, phényle, (C₁-C₆)-alkyle, (C₁-C₆)-alkyloxy, hydroxy-(C₁-C₆)-alkyle, (C₁-C₆)-alkylamino ou di-(-C₁-C₆)-alkylamino.

L'invention a plus particulièrement pour objet les composés de formule (I) tels que définis plus haut dans lesquels A est un groupement -CH₂-, B est un groupement -CH₂₋CH=CH- ou -CH₂-Cyclopropyle-, Ar¹ représente un phényle et Ar² représente un phénylène ainsi que leurs sels d'addition physiologiquement acceptables.

L'invention a plus particulièrement pour objet les composés de formule (I) tels que définis plus haut répondant à la structure : 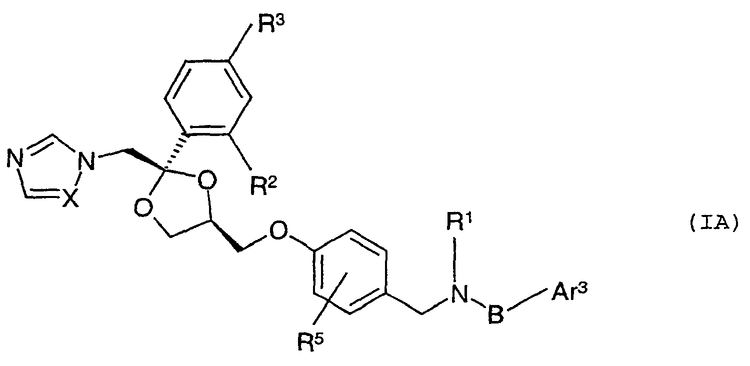
dans laquelle, B, X, Ar³, R⁵ et R¹ sont tels que définis plus haut et R² et R³ représentent un atome de chlore ou de fluor ainsi que leurs sels d'addition physiologiquement acceptables.

L'invention a plus particulièrement pour objet les composés de formule (I) ou (IA) telles que définis plus haut dans lesquelles R₂ et R₃ sont des atomes de fluor ou de chlore, X représente CH ou N et Ar³ représente un groupement phényle non substitué ou substitué par R⁸ tel que défini précédemment, ainsi que leurs sels d'addition physiologiquement acceptables.

L'invention a tout particulièrement pour objet les composés de formule (I) ou (IA) telle que définies précédemment dans lesquelles, R¹ est un atome d'hydrogène ou un groupement méthyle, ou éthyle non substitué ou substitué par un groupement F, OH, NH₂, (C₁-C₆)-alkyloxy, (C₁-C₆)-alkylamino, di-(C₁-C₆)-alkylamino, pyrrolidino ou 2-oxo-pyrrolidino ainsi que leurs sels d'addition physiologiquement acceptables.

L'invention a tout particulièrement pour objet les composés de formule (IA) telles que définies précédemment dans laquelle Ar³ est un phényle non substitué ou substitué par R⁸ représentant un radical -Cl, -F, CN, -CF₃, -OCF₃, -OH,
- NH₂, (C₁-C₆)-alkyloxy, (C₁-C₆)-alkylamino, di-(C₁-C₆)-alkylamino ou un hétérocycle choisi parmi :

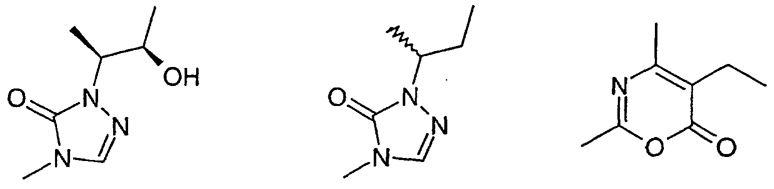

L'invention a tout particulièrement pour objet les composés suivants :
- cis-4-[[2-(2,4-dichlorophényl)-2-(1H-imidazol-1-ylméthyl)-1,3-dioxolan-4-yl]méthoxy]-N-méthyl-N-(3-phényl-2(E)-propényl)-benzèneméthanamine
- 4-[[2-(2,4-dichlorophényl)-2-(1H-imidazol-1-ylméthyl)-1,3-dioxolan-4-yl]méthoxy]-N-méthyl-N-[3-(4-chloro-phényl-2(E)-propényl]-1-benzeneméthanamine
- cis-4-[3-[[[4-[[2-(2,4-dichlorophényl)-2-(1H-imidazol-1-ylméthyl)-1,3-dioxan-4-yl]méthoxy]phényl]méthyl] méthylamino]-1(E)-propényl]-phénol
- cis-4-[[2-(2,4-dichlorophényl)-2-(1H-imidazol-1-ylméthyl)-2,3-dioxolan-4-yl]méthoxy]-N-(3-phényl-2(E)-propényl)-benzèneméthanamine
- Phosphate et trifluoroacétate de Cis-4-[3-[[[4-[[2-(2,4-dichlorophényl)-2-(1H-imidazol-1-ylméthyl)-1,3-dioxan-4-yl]méthoxy]phényl]méthyl]méthylamino]-1(E)-propényl]-phénol
- 4-[[2-(2,4-dichlorophényl)-2-(1H-imidazol-1-ylméthyl)-1,3-dioxolan-4-yl]méthoxy]-N-méthyl-N-(3-phényl-2(E)-propényl)-1-naphtalèneméthanamine
- cis-4-[[2-(2,4-dichlorophényl)-2-(1H-imidazol-1-ylméthyl)-1,3-dioxolan-4-yl]méthoxyl-N-(3-phényl-2(E)-propényl)-4-chloro-benzèneméthanamine
- Trifluoroacétate de cis-N-(2-aminoéthyl)-4-[[2-(2,4-dichlorophényl)-2-(1H-imidazol-1-ylméthyl)-1,3-dioxolan-4-yl]méthoxy]-N-(3-phényl-2(E)-propényl)-benzèneméthanamine
- cis-N-(2-aminoéthyl)-N-[3-(4-chlorophényl)-2(E)-propényl]-4-[[2-(2,4-dichlorophényl)-2-(1H-imidazol-2-ylméthyl)-1,3-dioxolan-4-yl]méthoxyl-benzèneméthanamine
- cis-2-[[[4-[[2-(2,4-dichlorophényl)-2-(1H-imidazol-1-ylméthyl)-1, 3-dioxolan-4-yl]méthoxy]phényl]méthyl](3-phényl-2(E)-propényl)aminol-éthanol.

L'invention a également pour objet un procédé de préparation des composés de formule (I) caractérisé en ce que l'on soumet un composé de formule (II) : 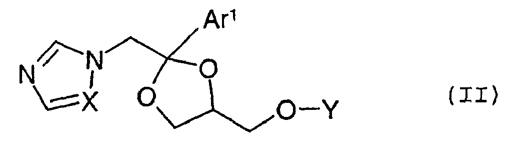
dans laquelle Y représente un groupe partant après substitution nucléophile tel que mésylate ou tosylate et les autres substituants conservent leur signification précédentes à l'action, en présence d'une base, d'un composé de formule (III)

HO-Ar²-A-N(R¹)-B-Ar³ (III)

dans laquelle Ar², A,R¹,B et Ar³ conservent leur précédente signification, pour obtenir le composé de formule (I) correspondant.

Cette réaction s'effectue dans les conditions classiques de substitution nucléophile du type R-OH + R'-OTs --> R-O-R' connues de l'homme du métier, Ts étant un groupe Tosyle. La base utilisée peut être notamment l'hydrure de sodium et le solvant peut être le DMF.

Les composés de formule (II) utilisés comme produits de départs sont des produits connus d'une façon générale, notamment lorsque Ar¹ est un phényle. Ils peuvent être préparés selon le procédé indiqué dans J. Med. Chem. (1979) 22(8) 1003.

Certains composés de formule (III) (R¹ = Me) sont aisément accessibles. Ils peuvent être préparés comme indiqué dans le schèma ci-dessous ou dans la partie expérimentale. 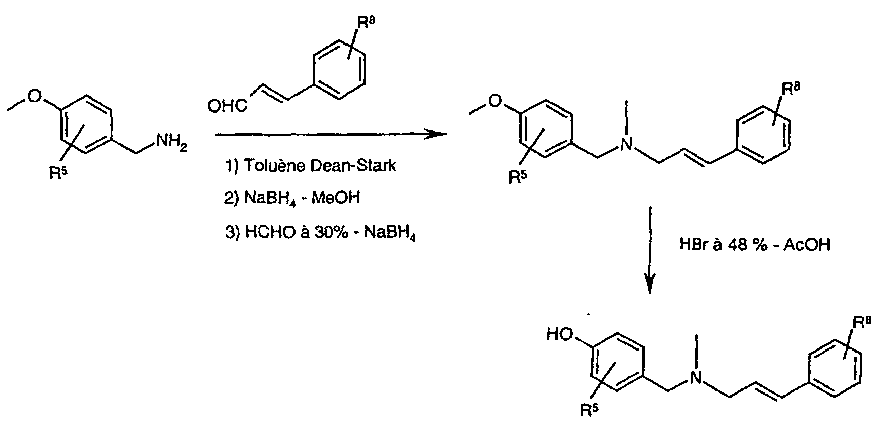 A titre de variante de procédé, on fait réagir le composé de formule (II) avec un aryle de formule (III') HO-C₄H₆-CHO en présence d'une base, le phényle étant non substitué ou substitué par R⁵, afin d'obtenir un composé de formule (IIa) : 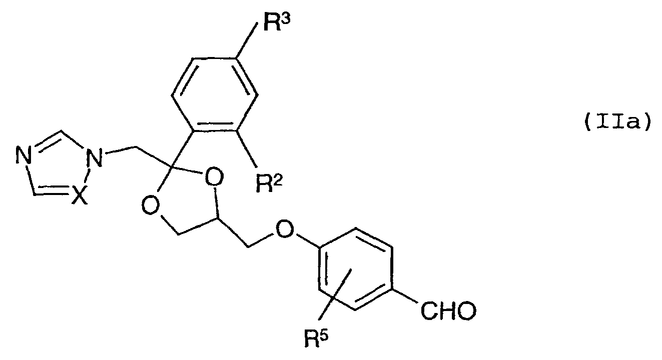 que l'on fait réagir avec une amine R¹-NH₂, R¹ étant tel que défini précédemment, suivi d'une réaction de réduction en présence d'un agent réducteur tel que NaBH₃CN, afin d'obtenir l'amine de formule (IIb) : 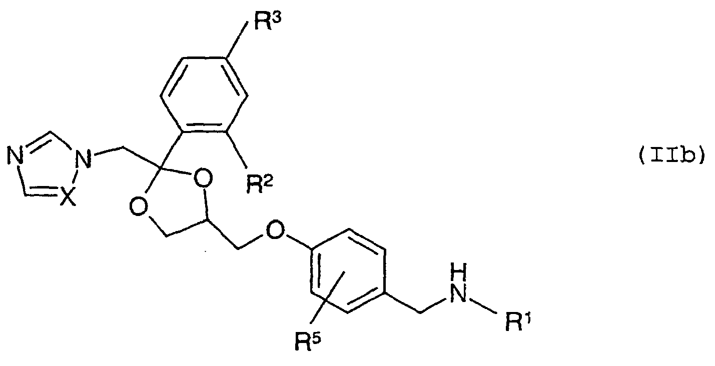 que l'on fait réagir
- soit avec un dérivé de formule :

   OHC-CH=CH-C₆H₄-R⁸ ou OHC-(Cyclopropyle)-C₆H₄-R⁸

   suivi d'une réaction de réduction en présence d'un agent de réduction tel que NaBH₃CN ou pyridine.BH₃
- soit avec un composé de formule :

   AcO-CH₂-CH=CH-C₆H₄-R⁸

   en présence d'un dérivé du palladium
   afin d'obtenir les composés de formules (IAA) et (IAB) suivantes :

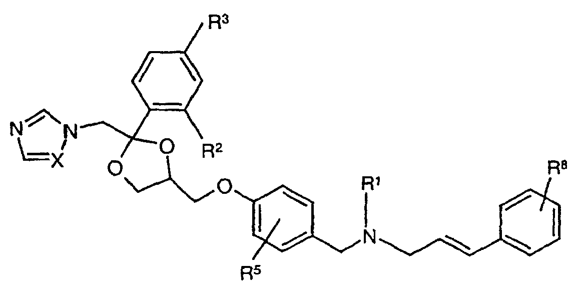

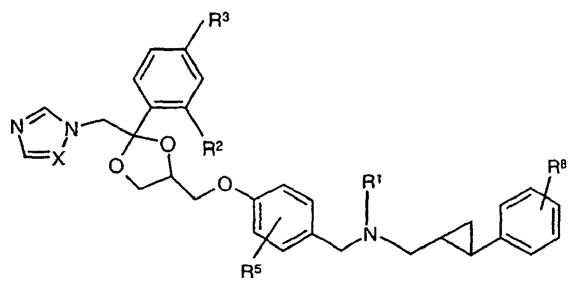

La première réaction d'amination réductrice mettant en jeu l'aldehyde (IIa) s'effectue de préférence en présence de réactifs tel que le NaBH₃CN dans le méthanol ou pyridin.BH₃. La deuxième réaction d'amination réductrice mettant en jeu l'amine (IIc) avec un dérivé de trans cinnamaldehyde, s'effectue également de préférence en présence de NaBH₃CN dans le méthanol. La réaction mettant en jeu l'amine (IIc) avec un acétate allylique s'effectue en présence d'un dérivé du palladium, par exemple en milieu acétonitrile/eau (tppts/Pd(OAc)₂).

De préférence, on fait réagir le composé de formule (IIa) avec une amine de formule R'¹-(CH₂)₂-NH₂, R'¹ représentant un groupement F, OH, une amine ou une alkylamine lesquelles étant convenablement protégées (telle que NHCO₂tbu pyrrolidino, 2-oxo-pyrrolidino), ou une dialkylamine afin d'obtenir un composé de formule (IIc), en présence d'un agent réducteur tel que NaBH₃CN : 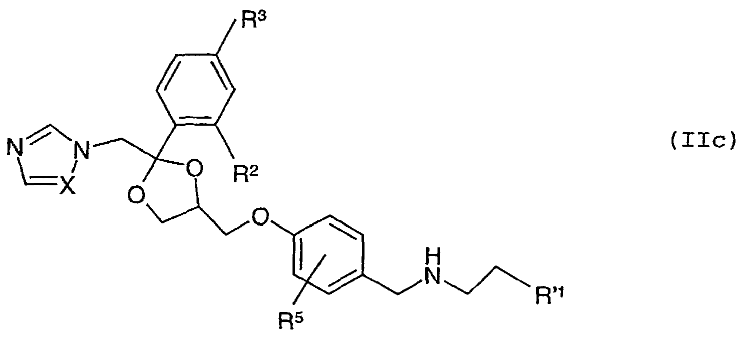 que l'on fait réagir avec un aldéhyde conjuguée tel que définie précédemment (OHC-CH=CH-C₄H₆-R⁸), en présence d'un agent réducteur tel que NaBH₃CN, afin d'obtenir un composé de formule (TAR)

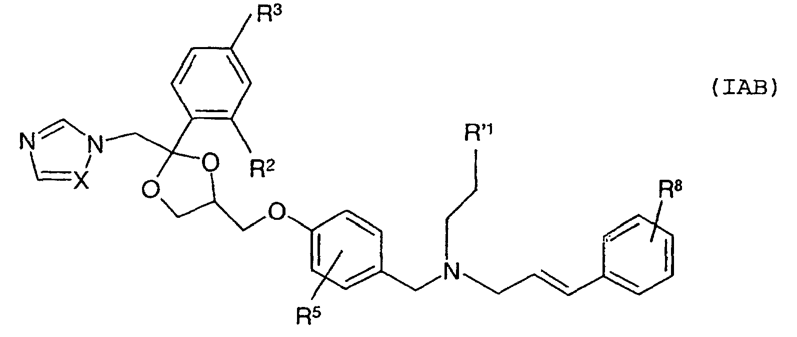

Les composés de départs de formule (II) ou (III) peuvent être préparés selon des procédés décrits dans la littérature ou encore sont accessibles par analogie. La préparation des composés de formule (II) est décrite dans Eur. J. Med. Chem. (1995) 30, 617-626 ou J. Heterocyclic Chemistry (1990), 27 2053, étant entendu que la présente invention n'est pas restreinte à ces synthèses ou à ces produits de départ. Il n'y a pas de difficulté majeure pour l'homme du métier de prévoir des modifications des synthèses décrites dans notre demande pour la préparation d'autres composés de formule (I) selon l'invention.

Les composés de formule (I) sont des composés ayant une activité pharmacologique et peuvent ainsi être utilisés à titre de médicaments notamment comme antifongiques

La présente invention a donc pour objet les composés de formule (I) et/ou leurs sels physiologiquement acceptables et/ou leurs prodrugs à titre de médicament.

Les composés de formule (I) ainsi que leurs sels physiologiquement acceptables et leurs prodrugs peuvent être administrés aux animaux, de préférence aux mammifères et en particulier aux êtres humains comme médicaments à titre thérapeutique ou prophylactique.

Les composés de formule (I) présentent d'intéressantes propriétés antifongiques. Ils sont notamment actifs sur *Candida albicans* et autres *Candidas* comme *Candida glabrata, krusei, tropicalis, pseudotropicalis* et *parapsilosis,* sur *Aspergillus, Aspergillus flavus,* *Aspergillus niger,* Cryptococcus *néoformans, Microsporum canis, Trichophyton rubrun, Trichophyton mentagrophyte.*

Les composés de formule (I) peuvent être utilisés en tant que médicament chez l'homme ou l'animal, pour lutter notamment contre les candidoses digestives, urinaires, vaginales ou cutanées, les cryptococcoses, par exemple les cryptococcoses neuroméningées, pulmonaires ou cutanées, les aspergilloses bronchopulmonaires et pulmonaires et les aspergilloses invasives de l'immunodéprimé.

Les composés selon l'invention peuvent être utilisés également dans la prévention des affections mycosiques chez les déprimés immunitaires congénitaux ou acquis.

Les composés de l'invention ne sont pas limités à une utilisation pharmaceutiques. Ils peuvent être également utilisés comme fongicides dans d'autres domaines que pharmaceutiques.

L'invention a donc pour objet à titre de médicaments antifongiques, les composés de formule (I).

L'invention a également pour objet l'application d'entités chimiques présentant à une extrémité les groupements suivants : 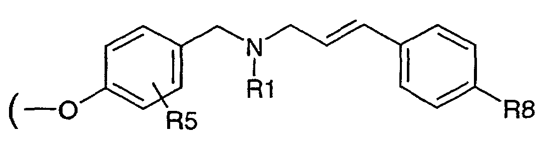 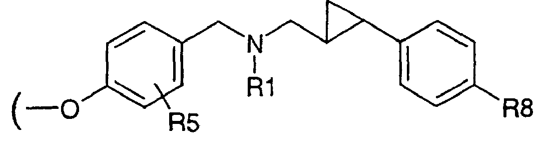 et à l'autre extrémité un groupement pharmacophore ayant une activité fongicide, par exemple un dérivéd'azole ou de triazole tel que défini précédemment, pour la préparation de médicaments présentant une activité antifongiques.

Les composés selon l'invention peuvent être administrés tels quels ou en mélange avec un ou plusieurs autres composés de formule (I) ou encore sous la forme d'une préparation pharmaceutique (composition pharmaceutique) qui permet une administration entérale ou parentérale et qui renferme à titre de composé actif une dose efficace d'au moins un composé de formule (I) et/ou ses sels physiologiquement acceptables et/ou ses prodrugs ainsi que des supports et/ou additifs courants et pharmaceutiquement inertes.

Les compositions pharmaceutiques selon l'invention permettent une administration entérale ou parentérale qui renferment à titre de composé actif une dose efficace d'au moins un composé de formule (I) et/ou ses sels physiologiquement acceptables et/ou ses prodrugs ainsi qu'un ou plusieurs supports pharmaceutiquement inertes, et/ou un ou plusieurs additifs usuels.

L'invention a donc pour objet les compositions pharmaceutiques renfermant un composé de formule (I) tel que défini précédemment ainsi qu'un véhicule.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire, par voie parentérale, par exemple sous forme de solutions injectables ou d'infusions, de microcapsules ou d'implants, par voie percutanée, par exemple sous la forme de pommade, de solutions, de pigments ou de colorants, par voie transdermique sous forme de patches ou par d'autres voies telles que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I) et/ou leurs sels physiologiquement acceptables et/ou leurs prodrugs. Pour la production de pilule, de comprimés, de comprimés enrobés et de capsule en gélatine dure, il est possible d'utiliser par exemple, du lactose, de l'amidon de maïs ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les microcapsules ou les implants sont par exemple les copolymères d'acide glyoxilique et d'acide lactique. Les préparations pharmaceutiques contiennent normalement de 0,5% à 90% en poids de composés de formule (I) et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants. Elles peuvent également contenir deux ou plusieurs composés de formule (I) et/ou leurs sels physiologiquement acceptables et/ou leurs prodrugs. En outre, en plus d'au moins un ou plusieurs composés de formule (I) et/ou leurs sels physiologiquement acceptables et/ou leurs prodrugs, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs.

La présente invention a donc plus particulièrement pour objet un composé de formule (I) et/ou ses sels physiologiquement acceptables et/ou ses prodrugs tels que définis plus haut à titre de médicament ayant une activité antifongique.

La présente invention a également pour objet l'utilisation des composés de formule (I) et/ou leurs sels physiologiquement acceptables et/ou leurs prodrugs tels que définis plus haut pour la préparation de médicaments antifongiques.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,01 à 100mg/kg et de préférence de 0,1 à 50 mg/kg, en particulier de 0,1 à 5 mg/kg.

Dans le cas d'une administration par voie intraveineuse, la dose quotidienne varie approximativement de 0,01 à 100 mg/kg et de préférence de 0,05 à 10 mg/kg. La dose quotidienne peut être divisée, en particulier dans le cas de l'administration de grande quantité de principe actif, en plusieurs, par exemple 2,3 ou 4 parts. Le cas échéant, en fonction du comportement individuel, il peut être nécessaire d'administrer les différentes doses de manière croissante ou décroissante.

Les composés de formule (I) et leurs sels peuvent également être utilisés comme intermédiaires pour la préparation d'autres composés, en particulier d'autres agents actifs, qui sont accessibles à partir des composés de formule (I), par exemple par modification ou introduction de radicaux ou de groupes fonctionnels.

Enfin, l'invention a pour objet à titre de composés intermédiaires des procédés tels que définis précédement, les composés de formules (IIa), (IIb), et (IIc).

### Exemples

Les produits ont été identifiés par spectre de masse (MS), infrarouge (IR) et/ou spectre RMN. Les composés ont été purifiés par chromatographie en phase normale (notamment en présence du mélange CH₂Cl₂/MeOH) ou en phase inverse (en présence d'acide acétique ou trifluoroacétique). Les composés de formule (I) purifiés en utilisant un éluant qui contient par exemple de l'acide trifluoroacétique, et qui ensuite sont séchés ou dans lesquels, lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

### Abbrévations/noms chimiques éventuellement employés:

AcOEt : acétate d'éthyle ; DMF : diméthylformamide ; HOBt : 1-hydroxybenzotriazole hydrate, MeOH : méthanol ; TEA : triéthylamine ; TFA : acide trifluoroacétique ; THF : tétrahydrofurane ; MCPBA : acide méta-chloroperoxybenzoique ; DBU : 1,8-diazabicyclo[5.4.0]undec-7-ene; APTS : acide paratoluènesulfonique ; DPPA : diphénylphosphorylazide ; DMSO : diméthylsulfoxyde ; Pd/C Palladium sur charbon ; Boc : terbutoxycarbonyl ; CBz : benzyloxycarbonyl ; DCC 1,3-dicyclohexylcarbodiimide ;
IR : Infrarouge ; RMN : Résonnance Magnétique Nucléaire ; SM : Spectre de Masse ; ESP : Electrospray mode positif ; ep. : Épaulement ; F : fort ; s : singulet ; d : doublet ; t : triplet ; quad : quadruplet ; quint : quintuplet ; 1 : large ; m : multiplet ou massif; J : constante de couplage ; Rf : facteur de rétention (chromatographie).

Les spectres RMN ci-dessous ont été interprétés et les hydrogènes aromatiques sont identifiées ainsi

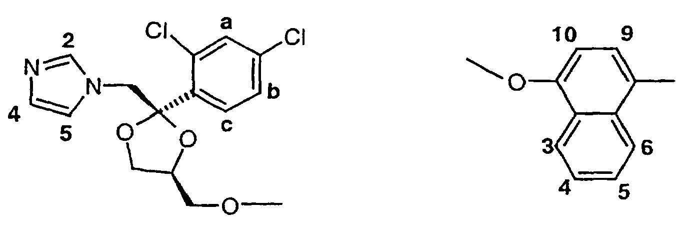

### Préparation 1 : 4-hydroxy-N-méthyl-N-(3-phényl-2(E)-propényl)-benzèneméthanamine (P1)

### Stade a) : 4-méthoxy-N-méthyl-N-(3-phényl-2(E)-propényl)-benzèneméthanamine

On chauffe au reflux pendant 2 heures 30 minutes une solution constituée de trans-cinnamaldehyde (Jansen, d=1,048, 13,2 g, 0,1 mol) et de 4-méthoxybenzylamine (Fluka, d=1, 057, 13,7 g, 0,1 mol) dans 250 ml de toluène, tout en éliminant l'eau formée au cours de la réaction à l'aide d'un « Dean-Starck », puis évapore sous pression réduite le toluène. Le résidu obtenu (base de Schiff) est ensuite solubilisé dans 150 ml de méthanol puis on réduit la base de Schiff en ajoutant à 40°C 3,8 g de NaBH₄. On ajoute enfin au milieu réactionnnel 81 ml de formaldehyde à 37% (réaction d'amino réduction), porte le mélange au reflux pendant 30 minutes et agite une nuit à température ambiante. Après évaporation du méthanol, on reprend le résidu avec du dichlorométhane, lave deux fois à l'eau et une fois avec une solution aqueuse saturée en NaCl, sèche sur MgSO₄ filtre et évapore sous pression réduite jusqu'à obtention d'un extrait sec que l'on purifie par chromatographie sur silice en éluant avec un mélange CH₂Cl₂/AcOEt 70/30. On obtient 9,07 g de produit attendu cristallisé. Rf 0,20 CH₂Cl₂ AcOEt 70/30 RMN ¹H (300MHz CDCl₃) 2,23 (s, 3H, CH₃-N) ; 3,18 (dl, 2H, N-CH₂-CH=CH-Ph) ; 6,31 (td, J= 16; 6,5 Hz, N-CH₂-CH=CH-Ph) ; 6,54 (d, 1H, J = 16 Hz, N-CH₂-CH=CH-Ph) ; 3,49 (s, 2H, Ph-CH₂₋N) ; 3,80 (s, 3H, Ph-O-CH₃) ; 6,86 et 7, 25 AA' BB' ; 7,31 (tl, 2H, H méta) ; 7,38 (dl, 2H, H ortho) ; 7,24 (masqué 1H, H para).

### Stade b) : 4-hydroxy-N-méthyl-N-(3-phényl-2(E)-propényl)-benzèneméthanamine

A une solution de produit préparé au stade précédent (1 g, 3,74 mmol) dans 20 ml d'acide acétique, on ajoute 20 ml d'acide bromhydrique à 48 % et chauffe au reflux pendant 5 heures et 30 minutes. Après évaporation sous pression réduite en entrainant l'eau avec de l'acétate d'éthyle, on obtient un extrait sec que l'on purifie par chromatographie sur silice en éluant avec le mélange CH₂Cl₂/MeOH 95/5 pour obtenir 660 mg de produit attendu. Rf 0,46 CH₂Cl₂/MeOH 95/5.

### Préparations 2 et 3 : 4-[cis-[2-(2,4-dichlorophényl)-2-(1H-imidazol-1-ylméthyl)-1,3-dioxolan-4-yl]méthoxy]-N-méthylbenzèneméthanamine

### Stade a) : 4-[cis-[2-(2,4-dichlorophényl)-2-(1H-imidazol-1-ylméthyl)-1,3-dioxolan-4-yl]méthoxy]-benzaldéhyde (P2)

A une suspension de NaH (1,723 g) dans le DMF (150 ml), on ajoute une solution de 4 -hydroxybenzaldehyde (4,59 g, 0,0376 mol) dans 80 ml de DMF et agite 30 minutes à température ambiante. On ajoute ensuite 20 g de 4-méthyl-benzènesulfonate de [cis-[2-(2,4-dichlorophényl)-2-(1H-imidazol-1-ylméthyl)-1,3-dioxolan-4-yl]méthyle] et chauffe une nuit à 90 °C. Après être revenu à température ambiante, on verse sur 500 ml d'eau et extrait avec 4 fois 300 ml de dichlorométhane, lave les phases organiques avec de l'eau saturée en NaCl, sèche la phase organique sur MgSO4, filtre et évapore sous pression réduite jusqu'à obtention d'un extrait sec que l'on reprend avec 40 ml d'éther. On observe la cristallisation. Les cristaux sont essorés, rincés et lavés avec 4 fois 20 ml d'éther. On obtient 14,48 g de produit attendu (ainsi que 5,84 g de produit récupéré dans les liqueurs-mères). Rf 0,18 CH₂Cl₂/MeOH 98/2.
RMN 300 MHz CDCl₃
3,35 (dd) et 3,77 (dd) 2H , 3,80 (dd) et 3,91 (dd) 2H : O-CH₂₋CH-CH₂-O; 4,41 (m, 1H O-CH₂-CH-CH₂-O); 4,45 et 4,55 (AB, 2H, N-CH₂-Cq) ; 6,42 et 7,85 (AA'BB') 9,90 (s, 1H, CHO) ; 7,03 (dl, 2H, H4 et H5) ; 7,29 (dd, 1H, Hb) ; 7,49 (d, 1H, Ha) ; 7,61 (d, 1H, Hc) ; 7,70 (s, 1H, H2).

### Stade b) : 4-[cis-[2-(2,4-dichlorophényl)-2-(1H-imidazol-1-ylméthyl)-1,3-dioxolan-4-yl]méthoxy]-N-méthyl-benzèneméthanamine (amino-réduction) (P3)

A une solution du produit P2 préparé au stade précédent (5,70 g ; 0,0131 mol) dans le méthanol (170 ml), on ajoute le chlorhydrate de méthylamine (8,833 g, 0,131 mol) et du NaBH₃CN (1,654 g, 0,026 mol) et agite 19 heures. On évapore sous pression réduite jusqu'à obtention d'un extrait sec et reprend avec un mélange eau/CH₂Cl₂/NaOH 2N (50 ml/20 ml/70 ml), reextrait la phase aqueuse avec 70 ml de dichlorométhane, lave avec de l'eau saturée en NaCl, sèche sur MgSO4, filtre et évapore sous pression réduite pour obtenir 6,1 g de produit brut attendu que l'on purifie par chromatographie sur silice en éluant avec un mélange CH₂Cl₂/MeOH 95/5 puis CH₂Cl₂/MeOH 87/13 auquel on a ajouté 1 % de TEA. On obtient 4,2 g de produit pur attendu. Rf 0,46 CH₂Cl₂/MeOH 90/10. RMN (300 MHz, CDCl₃)
2,44 (s, 3H, N-CH₃) ; 3,33 (dd) et 3,76 (dd) 2H , 3,73 (dd) et 3,88 (dd) 2H : : O-CH₂-CH-CH₂-O; 4,35 (m, 1H, O-CH₂-CH-CH₂-O) ; 3,68 (s, 2H, N-CH₂-C_{q}) ; 4,45 (AB, 2H) ; 6,78 et 7, 23 (AA'BB') ; 7,46 (d, 1H, Ha) ; 7,26 (dd, 1H, Hb) ; 7,58 (d, 1H, Hc) ; 7,50 (s, 1H, H2) . 6,98 (d, 2H, H4 et H5).

### Préparation 4 : 3-[4-[(2-méthoxyéthoxy)méthoxy]phényl]-2(E)-propènal

### Stade a) Protection : 4-[(2-méthoxyéthoxy)méthoxy]-benzaldéhyde

A une solution de 4-hydroxybenzaldehyde (24,4 g, 0,2 mol) dans 500 ml l'acétonitrile , on ajoute la diisopropylamine (70 ml, 0,4 mol) et refroidit à environ 9°C. On ajoute ensuite le chlorure de MEM (40,30 ml, 0,4 mol en 30 minutes, laisse revenir à température ambiante pendant 1 heure et évapore le milieu réactionnel sous pression réduite jusqu'à obtention d'un résidu que l'on reprend avec 500 ml de dichlorométhane. La phase organique est lavée avec de l'acide chlorhydrique 2N (2 x 500 ml) puis avec de la soude aqueuse 1N (2 x 500 ml) et enfin avec de l'eau saturée en NaCl. La phase organique est ensuite séchée sur MgSO₄, filtrée et amenée à sec. On obtient 41,322 g de produit attendu (huile). Rf 0,32 Cyclohexane / acétate d'éthyle 70/30.
RMN 300 MHz CDCl₃
3,38 (s, 3H, CH₃-O-(CH₂)₂-O-CH₂-O) ; 3,57 (m) et 3,89 (m) 4H CH₃-O-(CH₂)₂-O-CH₂-O); 5,36 (s, 2H, CH₃-O-(CH₂)₂-O-CH₂-O)); 7,18 et 7,85 (AA'BB'); 4,92 (s, 1H, CHO).

### Stade b) Wittig : 3-[4-[(2-méthoxyéthoxy)méthoxy]phényl]-2(E)-propènoate d'éthyle

A une solution constituée de (diéthoxyphosphinyl)-acétate d'éthyle (15,8 ml, 0,0786 mol), et de LiBr à 99% (6,895g, 0,0786 mol) dans 100 ml de THF, on ajoute 11 ml de TEA, agite pendant 10 minutes puis ajoute le dérivé préparé plus haut (15 g, 0,0714 mol) dans le THF (55 ml). On agite 12 heures, filtre afin d'éliminer le sel de TEA puis évapore sous pression réduite jusqu'à obtention d'un extrait sec que l'on reprend avec du dichlorométhane (200 ml). On lave avec de l'acide chlorhydrique puis avec une solution aqueuse de NaCl saturée. La phase organique est séchée sur MgSO₄ filtrée et amenée à sec pour obtenir un produit brut que l'on purifie par chromatographie sur silice en éluant avec le mélange AcOEt/Cyclohexane 30/70. On obtient 13,02 g du produit attendu purifié. Rf 0,43 Cyclohexane/acétate d'éthyle 70/30. RMN 300 MHz CDCl₃
1,33 (t, 3H, CH₃-CH₂-O) ; 4,25 (q, 2H, CH₃-CH₂-O) ; 3,37 (s, 3H, CH₃-O-(CH₂)₂-O-CH₂-O) ; 3, 55 (m) et 3, 82 (m, 4H, CH₃-O-(CH₂) ₂-O-CH₂-O) ; 5,30 (s, 2H, CH₃-O-(CH₂) ₂-O-CH₂-O)) ; 6,32 (d, 1H) , 7,64 (d, 1H) : Ph-CH=CH-CO ; 7,05 et 7,47 AA'BB'.

### Stade c) réduction 3-[4-[(2-méthoxyéthoxy)méthoxy]phényl]-2(E)-propèn-1-ol

A une solution du dérivé préparé au stade précédent (10 g, 0,0357 mol) dans 100 ml de dichlorométhane refroidit à -60°C, on ajoute le DIBAL (65 ml à 1M dans le dichlorométhane puis 6,5 ml à 1,5 mol dans le toluène) et agite 1 heure à -60°C. On laisse ensuite la température revenir à -10°C après avoir ajouté 40 ml d'acétate d'éthyle et verse le milieu réactionnel sur une solution 1M de tartrate de potassium et de sodium (750 ml). On ajoute 550 ml de dichlorométhane, lave, sèche et amène à sec pour obtenir 8,734 g de produit brut que l'on purifie par chromatographie sur silice en éluant avec le mélange dichlorométhane/méthanol 98/2. On obtient 8,287 g du produit attendu purifié. Rf 0,14 Cyclohexane/acétate d'éthyle 70/30.
RMN 300 MHz CDCl₃
1,41 (tl, 1H, HO-CH₂-CH=CH-Ph) ; 4,30 (1, 2H, HO-CH₂-CH=CH-Ph) ; 6,25 (td, 1H, J = 6, 16 Hz, HO-CH₂-CH=CH-Ph) ; 6,56 (dl, 1H, J=16 Hz, HO-CH₂-CH=CH-Ph) ; 3,37 (s, 3H, CH₃-O-(CH₂)₂-O-CH₂-O); 3,56 (m) et 3,82 (m) 4H CH₃-O-(CH₂)₂-O-CH₂-O); 5,27 (s1, 2H, CH₃-O-(CH₂)₂-O-CH₂-O) ; 7,01 et 7,32 AA' BB' .

### Stade d) Oxydation : 3-[4-[(2-méthoxyéthoxy)méthoxy]phényl]-2(E)-propènal

On agite pendant une nuit le mélange constitué de (I) (8 g, 0,034 mol) et de MnO₂ (28,9 g, 0,34 mol) dans le dichlorométhane 80 ml, filtre et évapore sous pression réduite jusqu'à obtention de 7,668 g d'une huile correspondant au produit attendu. Rf 0,38 Cyclohexane/acétate d'éthyle 60/40.

### Préparation 5 : cis-4-[[2-(2,4-dichlorophényl)-2-(1H-imidazol-1-ylméthyl)-1,3-dioxolan-4-yl]méthoxy]-N-méthyl-1-naphtalèneméthanamine

### Stade a) Couplage : cis-4-[[2-(2,4-dichlorophényl)-2-(1H-imidazol-1-ylméthyl)-1, 3-dioxolan-4-yl]méthoxy] -1-naphtalènecarboxaldéhyde

On ajoute à température ambiante du 4-hydroxy-1-naphtalènecarboxaldéhyde (860 mg, 5 mmol) à 260 mg d'hydrure de sodium à 55 % dans l'huile de vaseline dans 20 cm3 de DMF, puis on ajoute 2,41 g de 4-méthyl-benzènesulfonate de [cis-[2-(2,4-dichlorophényl)-2-(1H-imidazol-1-ylméthyl)-1,3-dioxolan-4-yl]méthyle] et agite 6 heures à 60°C puis 12 heures à température ambiante. On verse le milieu réactionnel sur de l'eau glacée, extrait au dichlorométhane, lave avec de la soude 2N puis avec une solution saturée de NaCl, sèche, filtre puis évapore sous pression réduite jusqu'à obtention d'un produit brut que l'on purifie par recristallisation dans le cyclohexane. On obtient 1,58 g de produit attendu. F=160°C. Rf 0,15 Cyclohexane/acétate d'éthyle 5/5.
RMN CDCl₃ 300 MHz
3,49 (dd, 1H) et 3,94 (dd, 1H), 3,92 (dd) et 4,01 (dd) 2H : O-CH₂-CH-CH₂-O ; 4,59 (m, 1H, O-CH₂-CH-CH₂-O) ; 4,46 et 4,55 (AB, 2H, N-CH₂-Cq) ; 6,80 (d, 1H, H10) ; 7,93 (d, 1H, H9) ; 9,30 (d) et 8,19 (dl) 2H H3 et H6 ; 7,57 (td) et 7,70 (td) 2H H4 et H5 ; 9,31 ; 7,50 (d, 1H, Ha) ; 7,63 (d, 1H, Hc) ; 7,31 (dd, 1H, Hb) ; 7,00 (s1, 2H, H'4 et H'5) ; 7,59 (sl, 1H, H'2) ; 10,20 (s, 1H, CHO). Les H' correspondent aux hydrogènes de l'imidazole.

### Stade b) Formation de l'amine :cis-4-[[2-(2,4-dichlorophényl)-2-(1H-imidazol-1-ylméthyl)-1,3-dioxolan-4-yl] méthoxy]-N-méthyl-1-naphtalèneméthanamine

A une solution d'aldehyde préparé au stade précédent (960 mg, 2 mmol) dans 20 cm3 de méthanol et 8 cm³ de dichlorométhane, on ajoute le chlorhydrate de méthylamine (1,33 g, 20 mmoles) puis du cyanoborohydrure de sodium (124 mg, 2 mmoles) et agite 12 heures à température ambiante. On évapore ensuite sous pression réduite et purifie le résidu par chromatographie sur silice en éluant avec un mélange CH₂Cl₂/MeOH 9/1. On obtient en plusieurs fractions 240 mg de produit atendu, 150 mg de produit attendu partiellement salifié et 90 mg d'un mélange des deux. Rf 0,20 CH₂Cl₂/MeOH 90/10.
RMN CDCl₃ 300 MHz
3,12 (m, 2H) et 3,80 (m, 2H) : O-CH₂-CH-CH₂-O ; 4,40 (m, 1H, O-CH₂-CH-CH₂-O) ; 4, 34 (AB, 2H, N-CH₂-Cq) ; 4, 41 (AB, 2H, N-CH₂-Cq) ; 2,70 (s, 3H, N-CH₃) ; 7,46 (d, 1H, Ha) ; 7,28 (dd, 1H, Hb) ; 7,57 (d, 1H, Hc) ; 6,68 (sl, 1H) ; 6,82 (sl, 1H) ; 7,20 (sl, 1H) : H de l'midazole ; 7,54 (d, 1H), 6,27 (d, 1H) H10 et H9 ; 8,13 (d) et 7,50 (masqué) et 7,64 (tl) et 7,99 (d) 4H : H3, H4, H5 et H6.

### Exemple 1 : cis-4-[[2-(2,4-dichlorophényl)-2-(1H-imidazol-1-ylméthyl)-1,3-dioxolan-4-yl]méthoxy]-N-méthyl-N-(3-phényl-2(E)-propényl)-benzèneméthanamine

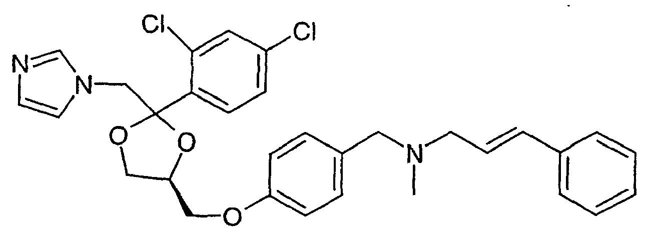

A une suspension constituée de 4-méthyl-benzènesulfonate de [cis-[2-(2,4-dichlorophényl)-2-(1H-imidazol-1-ylméthyl)-1,3-dioxolan-4-yl]méthyle] (180 mg, 0,372 mmol) (préparé selon J. Med Chem. (1979) Vol 22, n° 8 1003-1008), de P1 (104 mg, 0,410 mmol), de chlorure de tribenzylammonium (TEBAC) (10,4 mg), d'isobutylméthylcétone (MIBUC) (1,8 ml) et de carbonate de potassium (52 mg), on ajoute 1 goutte d'eau et chauffe à 80-90°C pendant 1 heure 15 puis 5 heures au reflux. On ajoute encore du 4-méthyl-benzènesulfonate de [cis-[2-(2,4-dichlorophényl)-2-(1H-imidazol-1-ylméthyl)-1,3-dioxolan-4-yl]méthyle] (78 mg, 0,161 mmol) et maintient le reflux pendant 2 heures 30 minutes puis on ajoute enfin une pointe de spatule de TEBAC et carbonate de potassium et maintien 13 heures 30 minutes au reflux. Après évaporation du milieu réactionnel, on purifie par chromatographie sur silice en éluant avec le mélange CH₂Cl₂/MeOH 98/2. On obtient deux fractions de 56 mg et 57 mg qui sont repurifiées pour obtenir 53 mg de produit attendu. Rf=0,40 CH₂Cl₂/MeOH 90/10. RMN ¹H (300MHz CDCl₃) 2,23 (s, 3H, CH₃-N) ; 3,19 (dl, 2H, N-CH₂-CH=CH-) ; 6,31 (td, 1H, J= 7, 16 Hz, N-CH₂-CH=CH-) ; 6,54 (dl, 1H, J= 16 Hz, N-CH₂-CH=CH-) ; 3,33 (dd) et 3,75 (m) 2H , 3,75 (m) et 3,89 (dd) 2H : O-CH₂-CH-CH₂-O; 4,36 (m, 1H, O-CH₂₋CH-CH₂-O) ; 7,26 (m, 1H, Hb) ; 3,51 (s1, 2H, N-CH₂-Cq) 4,40 et 4,51 (AB, 2H, Ph-CH₂-N) ; 6,78 et 7,25 (AA' BB', O-Ph) ; 6,99 (dl, 2H, H4 et H5) ; 7,52 (s1, 1H, H2) ; 7,58 (d, 1H, Hc) ; 7,47 (d, 1H, Ha) ; 7,39 (dl, 2H) , 7,32 (dl, 2H) , 7,26 (masqué) : H aromatiques.

### Exemple 2 : cis-4-[[2-(2,4-dichlorophényl)-2-(1H-imidazol-1-ylméthyl)-1,3-dioxolan-4-yl]méthoxy]-N-(3-phényl-2(E)-propényl)-4-chloro-benzèneméthanamine

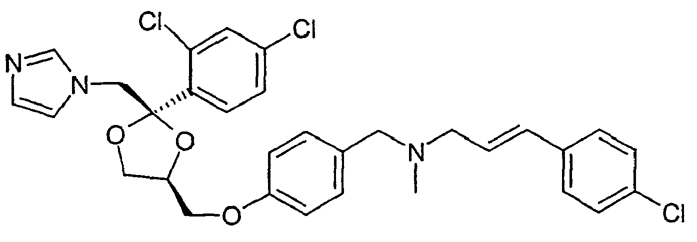

On mélange pendant une heure à température ambiante sous atmosphère d'azote 2 cm³ d'eau, 42 mg de TPPTS (sel de sodium du tris(3-sulfonatophényl)phosphine tétrahydraté, STREM CHEMICAL) et 18 mg d'acétate de palladium II puis ajoute une solution du dérivé aminé P3 (312 mg, 0,7 mmole) et d'acétate de para-chloro-(E)-cinnamyle (105 mg, 0,5 mmole dans l'acétonitrile (2 cm3). On agite 1 heure 30 minutes à 50°C, laisse revenir à température ambiante, ajoute de l'eau et extrait plusieurs fois au dichlorométhane, sèche sur MgSO4, filtre et évapore sous pression réduite pour obtenir le produit brut attendu que l'on purifie par chromatographie sur silice en éluant avec le mélange CH₂Cl₂/MeOH 90/10. On obtient 154 mg de produit purifié attendu. Rf 0,15 CH₂Cl₂/MeOH 9/1. RMN ¹H (300MHz CDCl₃) 2,32 (sl, 3H, CH₃-N) ; 3,30 (m, 2H, N-CH₂-CH=CH- ) ; 6,32 (dt, 1H, J = 16 et 7Hz, N-CH₂-CH=CH-) ; 6,52 (d, 1H, J = 16 Hz, N-CH₂-CH=CH-) ; 3, 30 (m) et 3,75 (m) 2H , 3,75 (m) et 3,89 (dd) 2H : O-CH₂-CH-CH₂-O; 4,36 (m, 1H, O-CH₂-CH-CH₂-O); 3,64 (s1, 2H, Cq-CH₂-N) ; 6,81 et 7,31 (AA'BB', Ph-O) ; 7,47 (d, 1H, Ha); 7,25 (masqué, 1H, Hb) ; 7,60 (d, 1H, Hc) ; 7, 31 (AA' BB', Ph) ; 7,53 (sl, H, H2) ; 6,92 (dl, 2H, H4 et H5).

### Exemple 3 : cis-4-[3-[[[4-[[2-(2,4-dichlorophényl)-2-(1H-imidazol-1-ylméthyl)-1,3-dioxan-4-yl]méthoxy]phényl]méthyl]méthylamino]-1(E)-propényl]-phénol

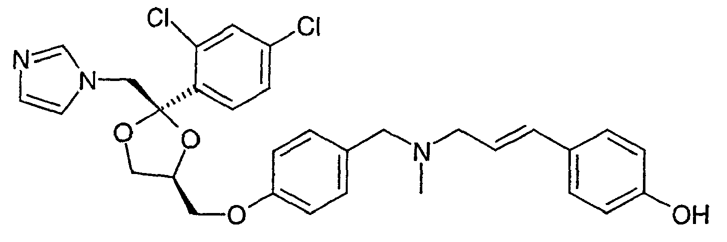

### Stade a) : cis-4-[[2-(2,4-dichlorophényl)-2-(1H-imidazol-1-ylméthyl)-1,3-dioxolan-4-yl]méthoxy]-N-[3-[4-[(2-méthoxyéthoxy)méthoxy]phényl]-2(E)-propényl]-N-méthylbenzèneméthanamine

A une solution de P3 (1,225 g, 0,0027 mol) dans le méthanol (40 ml) on ajoute P4 (1,232 g, 0,0052 mol), ajuste le pH à 6-7 par ajout de 100 µl d'acide acétique puis le NaBH₃CN à 95 % (373 mg, 0,00564 mol). Après 24 heures de réaction, on évapore sous pression réduite, et reprend le résidu obtenu avec le dichlorométhane (60 ml), lave, sèche et évapore à nouveau sous pression réduite jusqu'à obtention d'un extrait sec que l'on purifie par chromatographie sur silice en éluant avec le mélange dichlorométhane/Méthanol 95/5. On obtient 1,097 g du produit attendu purifié. Rf 0,47 dichlorométhane/Méthanol 93/7.
RMN 300 MHz DMSO
2,45 (s, 3H, N-CH₃) ; 3,23 (s, 3H, CH₃-O-(CH₂)₂-O-CH₂-O) ; 3,47 (m, 2H) et 3,73 (m, 2H) _{:} CH₃-O-(CH₂)₂-O-CH₂-O ; 5,25 (s, 2H, CH₃-O-(CH₂)₂-O-CH₂-O) ; 3,54 (d1, 2H, N-CH₂-CH=CH-Ph) ; 6,22 (td, 1H, J = 7, 16 Hz, N-CH₂-CH=CH-Ph) ; 6,66 (d1, 1H, J=16 Hz, N-CH₂-CH=CH-Ph) ; 3,94 (1, 2H, Ph-CH₂-N) ; 3,69 (m) et 3,90 (dd) 2H, 3,68 à 3,84 (m, 2H) : O-CH₂-CH-CH₂-O; 4,38 (m, 1H, O-CH₂-CH-CH₂-O) ; 4,54 (s, 2H, N-CH₂-Cq) ; 6,94 et 7,38 AA'BB', 7,01 et 7,42 AA'BB' : Ph-O ; 6,83 (s1) et 7,00 (m) 2H H4 et H5 ; 7,51 (s1, 1H, H2) ; 7,63 (d, 1H, Ha) ; 7,57 (d, 1H, Hc) ; 7,42 (m, 1H, Hb).

### Stade b) Déprotection : cis-4-[3-[[[4-[[2-(2,4-dichlorophényl)-2-(1H-imidazol-1-ylméthyl)-1,3-dioxan-4-yl]méthoxy]phényl]méthyl]méthylamino]-1(E)-propényl]-phénol

On agite pendant 3 heures à 0°C le mélange constitué de
(I) (1,028 g, 1,5 mmoles) dans 20 ml de dichlorométhane et 20 ml de TFA (acide trifluoroacétique), puis évapore sous pression réduite jusqu'à obtention d'un résidu qui est repris dans du dichlorométhane (60 ml). La phase organique est lavée, séchée et évaporée sous pression réduite jusqu'à obtention d'un extrait sec que l'on purifie par chromatographie sur silice en éluant avec le mélange dichlorométhane/éthanol 93/7. On obtient 425 mg du produit attendu purifié. Rf 0,16 dichlorométhane/Méthanol 93/7.
   RMN 300 MHz CDCl₃
   2,38 (s, 3H, N-CH₃) ; 3,69 (1, 2H, N-CH₂-Ph) ; 3,42 (dd) et 3,69 (masqué) 2H, 3,28 (masqué) et 3,75 (dd) 2H : O-CH₂-CH-CH₂-O ; 4,35 (m, 1H, O-CH₂-CH-CH₂-O) ; 3,30 (m, 2H, N-CH₂₋CH=CH-Ph) ; 5,90 (td, 1H, J = 7,5 ; 16 Hz, N-CH₂-CH=CH-Ph) ; 6,40 (d, 1H, J=16 Hz, N-CH₂-CH=CH-Ph) ; 4,52 et 4,43 (AB, 2H, N-CH₂-Cq) ; 6,76 et 7,26 (AA 'BB'), 6,80 et 7,09 (AA 'BB') 8H ; 7,63 (s1, 1H, H2) ; 7,64 (d, 1H, Hc) ; 7,49 (d, 1H, Ha) ; 7,29 (masqué Hb) ; 7,03 (m, 2H, H4 et H5).

### Exemple 4 : cis-4-[[2-(2,4-dichlorophényl)-2-(1H-imidazol-1-ylméthyl)-1,3-dioxolan-4-yl]méthoxy]-N-(3-phényl-2(E)-propényl)-benzèneméthanamine

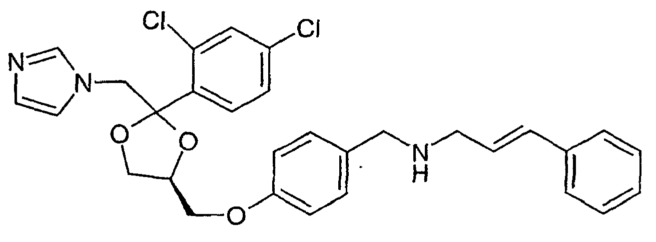

A une solution de P2 (433 mg, 1 mmole) et de chlorhydrate de 3-phénylallylamine (ref Sigma) (200 mg, 1,2 mmole) dans 12 cm3 de méthanol on ajoute du cyanoborohydrure de sodium (62 mg, 169 mmol) et agite une nuit à température ambiante. On évapore ensuite sous pression réduite, reprend le résidu huileux dans l'acétate d'éthyle, lave avec une solution de soude 2N, sèche sur MgSO₄, et amène à sec sous vide. On obtient 592 mg de produit brut que l'on purifie par chromatographie sur silice en éluant avec le mélange CH₂Cl₂/MeOH 9/1 auquel on a ajouté 1 % d'eau. On obtient 225 mg de produit pur attendu. Rf 0,20 CH₂Cl₂/MeOH 90/10 + 1 % eau.
3,33 (dd) et 3,76 (m) 2H, 3,75 (m) et 3,84 (dd) 2H : O-CH₂-CH-CH₂-O ; 4,36 (m, 1H, O-CH₂-CH-CH₂-O) ; 4,40 et 4,51 (AB, 2H, N-CH₂-Cq) ; 3,43 (dl, 2H, N-CH₂-CH=CH-Ph) ; 6,32 (td, 1H J = 16 et 6,5 Hz, N-CH₂-CH=CH-Ph) ; 6,55 (dl, 1H, J=16 Hz, N-CH₂₋CH=CH-Ph) ; 3,78 (sl, 2H, N-CH₂-Ph) ; 6,79 et 7,26 (AA 'BB', 4H, Ph-O) ; 6,98 (m, 2H, H4 et H5) ; 7,47 (d, 1H, Ha) ; 7,58 (d, 1H, Hc) ; 7,38 (dl, 2H, H ortho) ; 7,31 (tl, 2H, H méta) ; 7., 23 (masqué, 2H, H para et Hb) ; 7,51 (sl, 1H, H2).

### Exemple 5 : Phosphate de mono[cis-4-[3-[[[4-[[2-(2,4-dichlorophényl)-2-(1H-imidazol-1-ylméthyl)-1,3-dioxan-4-yl]méthoxy]phényl]méthyl]méthylamino]-1(E)-propényl]phényle] (sel de trifluoroacétate)

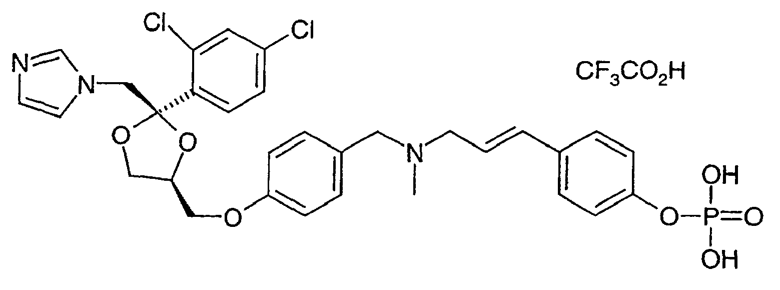

### Stade a) Phosphorylation : Phosphate de [cis-4-[3-[[[4-[[2-(2,4-dichlorophényl)-2-(1H-imidazol-1-ylméthyl)-1,3-dioxan-4-yl]méthoxy]phényl]méthyl]méthylamino]-1(E)-propényl]phényle] et de bis(phénylméthyl)

A une solution de (I) (400 mg, 0,689 mmol) dans le dichlorométhane (12 ml) refroidie à -5 °C, on ajoute 665 µl de CCl₄, 17 mg de DMAP, 480 µl de diisopropyléthylamine puis goutte à goutte du dibenzylphosphite (460 µl, 2,08 mmol). On agite 3 heures à 0°C puis lave le milieu réactionnel avec 30 ml de NaH₂PO₄ (1M) puis 10 ml de solution aqueuse saturée en NaCl. Les phases organiques sont ensuite séchées puis évaporées sous pression réduite jusqu'à obtention d'un extrait sec que l'on purifie par filtration sur silice en éluant avec le mélange dichlorométhane/MeOH 95/5. On obtient 234 mg de produit attendu. Rf 0,42 dichlorométhane/méthanol 93/7.

### Stade b) Déprotection : Phosphate de mono[cis-4-[3-[[[4-[[2-(2,4-dichlorophényl)-2-(1H-imidazol-1-ylméthyl)-1,3-dioxan-4-yl]méthoxylphényl]méthyl]méthylamino]-1(E)-propényl]phényle] (sel de trifluoroacétate)

On agite pendant 5 heures à température ambiante le mélange constitué de (I) (234 mg) dans 5 ml de dichlorométhane et 5 ml de TFA (acide trifluoroacétique), puis évapore sous pression réduite jusqu'à obtention d'un extrait sec que l'on purifie par HPLC sur Kromasil C18 10 µ en éluant avec le mélange acétonitrile/eau 40/60 (+ 0,03 % de TFA). On obtient 119 mg du produit attendu purifié. Rf 3,24 acétonitrile/eau 40/60.
RMN 300 MHz DMSO
2,65 (s, 3H, N-CH₃) ; 4,28 (sl, 2H, N-CH₂-Ph) ; 3,85 (dl, 2H, N-CH₂-CH=CH-Ph) ; 6,29 (td, 1H, J = 6,5 ; 16 Hz, N-CH₂-CH=C_{H-}Ph) ; 6, 80 (d1, 1H, J=16 Hz, N-CH₂-CH=CH-Ph) ; 4,76 et 4, 83 (AB, 2H, N-CH₂-Cq) ; 9,00 (sl, 1H, H2) ; 3,68 (dd) et 3,43 (dd) 2H, 3,71 (dd) et 3,87 (dd) 2H : O-CH₂-CH-CH₂-O ; 4,41 (m, 1H, O-CH₂-CH-CH₂-O) ; 6,96 et 7,46 AA'BB', 7,18 et 7,49 AA'BB' (8H) ; 7,54 (m, 3H, Hb, H4 et H5) ; 7,65 (d, 1H, Hc) ; 7, 74 (d, 1H, Ha) ; 9,97 (sl, 1H mobiles)

### Exemple 6 : cis-4-[[2-(2,4-dichlorophényl)-2-(1H-imidazol-1-ylméthyl)-1,3-dioxolan-4-yl]méthoxy]-N-méthyl-N-(3-phényl-2(E)-propényl)-1-naphtalèneméthanamine

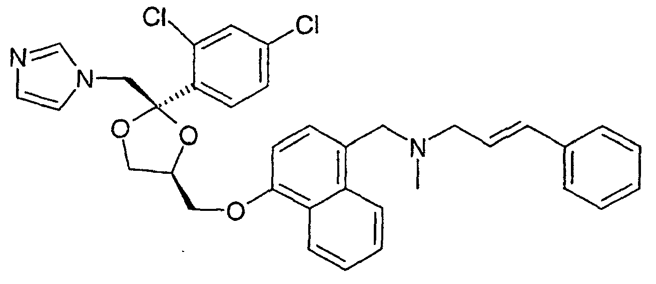

A un mélange constitué de P5 (240 mg, 0,48 mmole) et de trans-cinnamaldehyde (120 µl, 0,96 mmole) dans le méthanol (10 cm³) dont le pH est égal à environ 6 par addition d'acide acétique (15 µl), on ajoute le cyanoborohydrure de sodium (30 mg) et laisse réagir 12 heures sous agitation. On évapore ensuite sous pression réduite et purifie par chromatographie sur silice en éluant avec un mélange CH₂Cl₂/MeOH 93/7 pour obtenir 104 mg de produit attendu. Rf 0,20 CH₂Cl₂/MeOH 93/7. RMN CDCl₃ 300 MHz
2,28 (s, 3H, N-CH₃) ; 3,30 (dl, 2H, N-CH₂-CH=CH-Ph) ; 6,38 (tl, 1H, J = 7, 16 Hz, N-CH₂-CH=CH-Ph) ; 6,58 (dl, 1H J = 16 Hz, N-CH₂-CH=CH-Ph) ; 4,45 et 4,54 (AB, 2H, N-CH₂-Cq) ; 3,92 (sl, 2H, N-CH₂-Ph) ; 3,54 (dd) et 3,96 (dd) 2H, 3,88 (dd) et 3,98 (dd) 2H : O-CH₂-CH-CH₂-O ; 4,54 (m, 1H, O-CH₂-CH-CH₂-O ; 8,18 (dl, 1H) et 8,23 (dl, 1H) : H3 et H6 ; 7,49 (d, 1H, Ha) ; 7,60 (d, 1H, Hc) ; 6,65 (dl, 1H, H10) ; 7,37 (m, 1H, H9) ; 7,00 (s1, 2H) , 7,16 à 7,58 (m, 9H) : Hb, H2', H4', H5', H3, H6, H du phényle.

### Exemple 7 : cis-4-[[2-(2,4-dichlorophényl)-2-(1H-imidazol-1-ylméthyl)-1,3-dioxolan-4-yl]méthoxy]-N-[3-[4-(2-hydroxyéthoxy)phényl]-2(E)-propényl]-N-méthyl-benzèneméthanamine

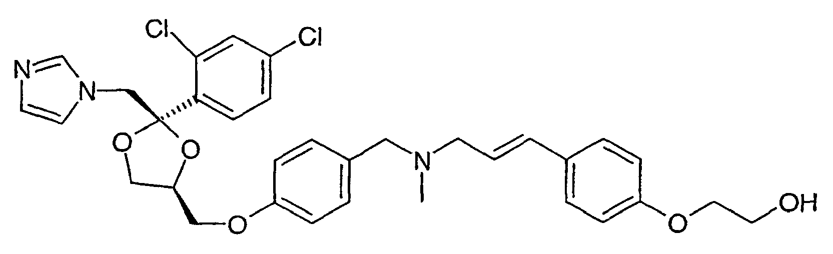

On agite une nuit à 80°C le mélange constitué du phénol préparé à l'exemple 3 (89 mg, 0,15 mmol), de K₂CO₃ (23 mg, 0,16 mmol) et de 1,3-dioxolan-2-one (68 mg, 0,77 mmol) dans 2 ml de DMF, puis dilue le mélange réactionnel avec 10 ml d'acétate d'éthyle. Après lavage et séchage, on évapore sous pression réduite pour obtenir 110 mg de produit brut que l'on purifie par chromatographie sur silice en éluant avec le mélange CH₂Cl₂/MeOH 93/7. On obtient 32 mg de produit pur attendu. Rf 0,30 CH₂Cl₂/MeOH 93,00/7,00.
3,33 (dd) et 3,76 (m) 2H , 3,75 (m) et 3,84 (dd) 2H : O-CH₂₋CH-CH₂-O ; 4,37 (m, 1H, O-CH₂-CH-CH₂-O) ; 3,97 (m, 2H), 4, 07 (m, 2H) : O-(CH₂)₂-O ; 4,41 et 4,52 (AB, 2H, N-CH₂-Cq) ; 6,97 (m, 2H, H4 et H5) ; 3,24 (sl, 2H, N-CH₂-CH=CH-Ph) ; 6,20 (td, 1H J = 16 et 7 Hz, N-CH₂-CH=CH-Ph) ; 6,51 (dl, 1H, J=16 Hz, N-CH₂-CH=CH-Ph) ; 2,28 (sl, 3H, N-CH₃) ; 3, 57 (s1, 2H, Ph-CH₂₋N) ; 6,79 et 7,28 (AA' BB'), 6,87 et 7,33 (AA ' BB') : 8H ; 7,26 (masqué, 1H, Hb) ; 7,59 (d, 1H, Hc) ; 7,47 (d, 1H, Ha) ; 7,51 (s1, 1H, H2).

### Exemple 8 : Trifluoroacétate de cis-N-(2-aminoéthyl)-4-[[2-(2,4-dichlorophényl)-2-(1H-imidazol-1-ylméthyl)-1,3-dioxolan-4-yl]méthoxy]-N-(3-phényl-2(E)-propényl)-benzèneméthanamine

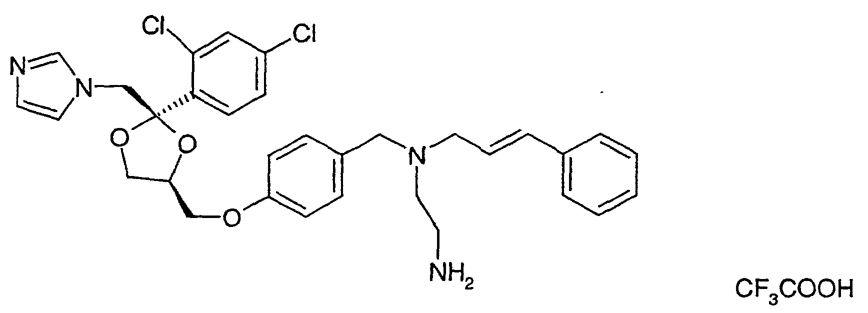

### Stade a) cis-[2-[[[4-[[2-(2,4-dichlorophényl)-2-(1H-imidazol-1-ylméthyl)-1,3-dioxolan-4-yl]méthoxy]phényl]méthyl]amino éthyl]-carbamate de 1,1-diméthyléthyle (P6)

A une solution de P2 (1 g, 2,30 mmol) dans le MeOH (30 ml) on ajoute la N-Boc-éthylènediamine (400 µl), ajuste le pH à environ 6 en ajoutant de l'acide acétique (200 µl) puis au bout de 15 minutes, on ajoute le NaBH₃CN (227 mg) et agite 18 heures à température ambiante. Le milieu réactionnel est ensuite filtré, et amené à sec. Le résidu est repris dans du dichlorométhane, la phase organique est lavée, sèchée puis évaporée sous pression réduite pour obtenir 1,3 g de produit brut que l'on purifie par chromatographie en éluant avec le mélange CH₂Cl₂/MeOH (9/1). On obtient 875 mg de produit attendu. Rf 0,16 CH₂Cl_{2/}MeOH 90/10.
RMN CDCl₃ 300 MHz
1, 43 (s, 9H, 0-C(CH₃)₃) ; 3,42 (dd) et 3,66 (dd) 2H, 3,78 (dd) et 3,89 (dd) 2H : O-CH₂-CH-CH₂-O ; 4,37 (m, 1H, O-CH₂-CH-CH₂₋0) ; 3,00 (m, 1H, Ph-CH₂-NH-CH₂-CH₂-NH) ; 3,00 (tl, 2H) ; 3, 94 (sl, 2H, Ph-CH₂-NH-CH₂-CH₂-NHCO) ; 3,35 (m, 2H, Ph-CH₂-NH-CH₂₋CH₂-NHCO) ; 5,42 (t1, 1H, Ph-CH₂-NH-CH₂-CH₂-NHCO ) ; 4,41 et 4,48 (AB, 2H, N-CH₂-Cq) ; 6,80 et 7,35 (AA' BB' 4H, O-Ph) : 7,28 (m, 1H, Hb) ; 7,47 (d, 1H, Ha) ; 7,58(d, 1Hc) ; 7,46 (masqué, 1H, H2) ; 6,93 (sl, 2H, H4 et H5)

### Stade b) cis-[2-[[[4-[[2-(2,4-dichlorophényl)-2-(1H-imidazol-1-ylméthyl)-1,3-dioxolan-4-yl]méthoxy]phényl]méthyl] (3-phényl-2(E)-propényl)amino]éthyl]-carbamate de 1,1-diméthyléthyle

A un mélange constitué de (I) (287 mg, 0,498 mmol) et de trans-cinnamaldehyde (69,2 µl) dans le méthanol (6 ml) on ajoute après 15 minutes, en maintenant un pH de l'ordre de 6, le NaBH₃CN (54 mg) et laisse réagir pendant 1 heure 10 minutes sous agitation. On évapore ensuite sous pression réduite et purifie par chromatographie sur silice en éluant avec un mélange CH₂Cl₂/MeOH 95/5 pour obtenir 135 mg de produit attendu. Rf 0,49 CH₂Cl₂/MeOH 95/5.
RMN CDCl₃ 300 MHz
1,43 (s, 9H, O-C(CH₃)₃) ; 2,62 (t1, 2H, Ph-CH₂-NH-CH₂-CH₂₋NHCO) ; 3,24 (tl, 2H, Ph-CH₂-NH-CH₂-CH₂-NHCO) ; 4,99 (s1, 1H, Ph-CH₂-NH-CH₂-CH₂-NHCO) ; 3,34 (dd) et 3,76 (dd) 2H , 3,75 (dd) et 3,90 (dd) 2H : O-CH₂-CH-CH₂-O ; 4,37 (m, 1H, O-CH₂-CH-CH₂-O) ; 4,41 et 4,53 (AB, 2H, N-CH₂-Cq) ; 3,60 (s1, 2H, Ph-CH₂-N) ; 3,27 (masqué, 2H, N-CH₂-CH=CH-Ph) ; 6,27 (td, 1H, J=7, 16 Hz, N-CH₂-CH=CH-Ph) ; 6,52 (dl, 1H, J=16 Hz, N-CH₂₋CH=CH-Ph) ; 6,79 et 7,25 (AA'BB' 4H, Ph-O) ; 6,99 (m, 2H, H4 et H5) ; 7,58 (m, 1H, Hc) ; 7,52 (s1, 1H, H2) ; 7,47 (d, 1H, Ha) ; 7,26 (m, 1H, Hb) ; 7,20 à 7,40 (m, 5H Ph)

### Stade c) Trifluoroacétate de cis-N-(2-aminoéthyl)-4-[[2-(2,4-dichlorophényl)-2-(1H-imidazol-1-ylméthyl)-1,3-dioxolan-4-yl]méthoxy]-N-(3-phényl-2(E)-propényl)-benzèneméthanamine

A une solution de (I) (130 mg, 0,178 mmol) dans le dichlorométhane (1,4 ml), on ajoute à 0 - 5°C, l'acide trifluoroacétique (3,4 ml) agite 20 minutes à cette température puis laisse évoluer à la température ambiante pendant 2 heures. On évapore sous pression réduite, reprend le résidu avec du dichlorométhane, lave, sèche et amène à sec pour obtenir 115 mg de produit brut que l'on purifie par HPCL sur Kramasil C18 en éluant avec le mélange acétonitrile/eau 60/40 auquel on a ajouté 0,03 % de TFA. On obtient 79 mg de produit pur attendu. Rf 3,68 acétonitrile/eau 60/40 auquel + 0,03 % de TFA
RMN CDCl₃ 300 MHz
4,57 et 4,67 (AB, 2H, N-CH₂-Cq) ; 4,00 (1, 2H, N-CH₂-CH=CH-Ph) ; 6,39 (1, 1H, J=7, 16 Hz, N-CH₂-CH=CH-Ph) ; 6,85 (d1, 1H, J=16 Hz, N-CH₂-CH=CH-Ph) ; 7,33 (masqué, 1H, Hb) ; 7,50 (d, 1H, Ha) ; 7,66 (dd, 1H, Hc) ; 6,76 et 7,37 (AA'BB' 4H, Ph-O) ; 3,88 (m) et 3,50 (m) et 3,47 (m) 4H : O-CH₂-CH-CH₂-O ; 4,40 (s1, 1H, O-CH₂-CH-CH₂-O) ; 3,41 (1) et 3,62 (1) 4H : N-(CH₂)₂-NH₂ ; 4,19 (1, 2H, N-CH₂-Ph) ; 8,66 (s, 1H, H2) ; 7,19, (s1) et 7,37 (s1) 2H : H4 et H5 ; 7,45 (m, 2H) et 7,36 (m, 3H) : H du phényle.

### Exemple 9 : cis-N-(2-aminoéthyl)-N-[3-(4-chlorophényl)-2(E)-propényl]-4-[[2-(2,4-dichloxophényl)-2-(1H-imidazol-1-ylméthyl)-1,3-dioxolan-4-yl]méthoxy]-benzèneméthanamine

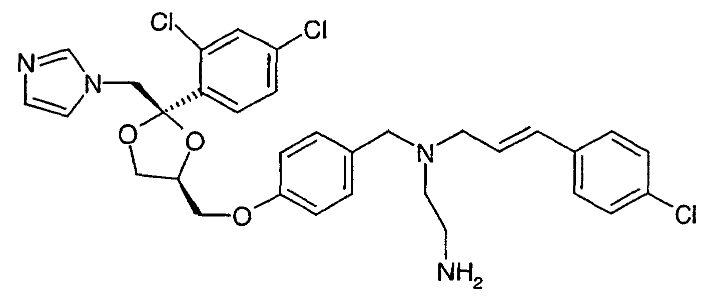

### Stade a) cis-[2-[[[[3-(4-chlorophényl)-2(E)-propényl]-4-[[2-(2,4-dichlorophényl)-2-(1H-imidazol-1-ylméthyl)-1,3-dioxolan-4-yl]méthoxy]phényl]méthyl]amino]éthyl]-carbamate de 1,1-diméthyléthyle

On agite à température ambiante pendant 1 heure le mélange constitué d'acétate de palladium (II) (7 mg) et de Tris(3-Sulfonatophényl)phosphine tétrahydrate de sodium (tppts, Strem Chemical) (52,6 mg) dans de l'eau déminéralisée (2 ml), puis ajoute une solution de Para-chloro-(E)-cinnamyl acétate (130 mg, 0,617 mol) et de P6 (500 mg) dans l'acétonitrile (2 ml). On agite 2 heurs à 50°C puis évapore l'acétonitrile et extrait la phase aqueuse restante avec du dichlorométhane. La phase organique est ensuite séchée, filtrée et évaporée sous pression réduite jusqu'à obtention d'un extrait sec ((650 mg) que l'on purifie par chromatographie sur silice en éluant avec le mélange CH₂Cl₂/MeOH (95/5) puis (98/2). On obtient 134 mg de produit attendu. Rf 0,63
CH₂Cl₂/MeOH (90/10).
RMN (CDCl₃) 300 MHz
1,43 (s, 9H, OC(CH₃)₃) ; 2,62 (s1, 2H, N-CH₂-CH₂-NH) ; 3,24 (1, 2H, N-CH₂-CH₂-NH) ; 3,26 (m, 2H, N-CH₂-CH=CH-Ph) ; 6,23 (td, 1H, J= 6,5 16 Hz, N-CH₂-CH=CH-Ph) ; 6,47 (dl, 1H, J = 16 Hz, N-CH₂-CH=CH-Ph) ; 3,31 (dd) et 3,74 (dd) 2H , 3,74 (dd) et 3,89 (dd) 2H : O-CH₂-CH-CH₂-O ; 4,36 (m, 1H, O-CH₂-CH-CH₂-O) 4,41 et 4,52 (AB, 2H, N-CH₂-Cq) ; 3,60 (s1, 2H, Ph-CH₂-N) ; 4,97 (s1, 1H, H mobile) ; 6,78 et 7,24 (AA'BB', 4H, Ph-O) ; 7,29 (m, 4H, H du phényle) ; 7,27 (m, 1H, Hb) ; 7,59 (d, 1H, Hc) ; 7,47 (d, 1, Ha) ; 6,99 (m, 2H, H4 et H5) ; 7,52 (sl, 1H, H2).

### Stade b) Déprotection : cis-N-(2-aminoéthyl)-N-[3-(4-chlorophényl)-2(E)-propényl]-4-[[2-(2,4-dichlorophényl)-2-(1H-imidazol-1-ylméthyl)-1,3-dioxolan-4-yl]méthoxy]-benzèneméthanamine

A une solution du dérivé préparé au stade précédent (130 mg, 0,178 mmol) dans le dichlorométhane (1,4 ml) on ajoute, à 0°C - 5°C, de l'acide trifluoroacétique (3,4 ml), agite 20 minutes à cette température puis laisse le milieu évoluer à la température ambiante pendant 2 heures. Après évaporation sous pression réduite, on reprend dans du dichlorométhane, lave avec de la soude 2N puis avec une solution saturée en NaCl. La phase organique est séchée, filtrée et amenée à sec. On obtient 108 mg de produit attendu. Rf 0,09 CH₂Cl₂/MeOH (85/15) .
RMN (CDCl₃) 300 MHz
2,78 (m, 2H, N-CH₂-CH₂-N) ; 2,54 (m, 2H, N-CH₂-CH₂-N) ; 3,39 (dd) et 3,60 (dd) 2H, 3,76 (dd) et 3,87 (dd) 2H : O-CH₂-CH-CH₂-O ; 4,37 (m, 1H, O-CH₂-CH-CH₂-O) ; 4,39 et 4,50 (AB, 2H, N-CH₂-Cq) ; 3,27 (d, 2H, N-CH₂-CH=CH-Ph) ; 6,26 (td, 1H J = 7, 16 Hz, N-CH₂-CH=CH-Ph) ; 6,48 (dl, 1H, J = 16 Hz, N-CH₂-CH=CH-Ph) ; 3,57 (sl, 2H, Ph-CH₂-N) ; 6,77 et 7,23 (AA'BB' , 4H, Ph-O) ; 6,96 (m, 2H, H4 et H5) ; 7,29 (m, 4H, H du phényle) ; 7, 47 (d, 1H, Ha) ; 7,43 (s1, 1H, H2) ; 7,60 (d, 1H, Hc) ; 7,27 (m, 1, Hb).

### Exemple 10: cis-2-[[[4-[[2-(2,4-dichlorophényl)-2-(1H-imidazol-1-ylméthyl)-1,3-dioxolan-4-yl]méthoxy]phényl]méthyl] (3-phényl-2(E)-propényl)arnino)-éthanol

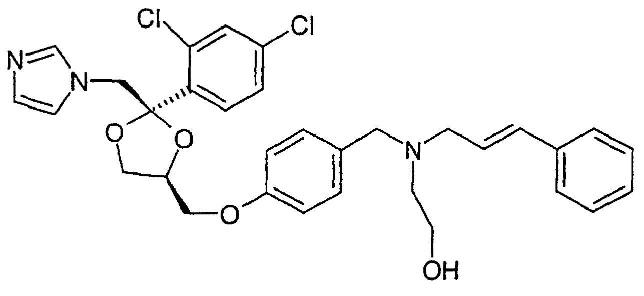

### Stade a) Formation de l'alcool : cis-2-[[[4-[[2-(2,4-dichlorophényl)-2-(1H-imidazol-1-ylméthyl)-1,3-dioxolan-4-yl]méthoxy]phényl]mëthyl]amino]-éthanol

On mélange P2 (1 g, 2,30 mmol) et de l'éthanolamine (153 µl) dans 30 ml de méthanol, ajoute de l'acide acétique (200 µl) en ajustant le pH à 5,5 puis après 15 minutes le NaBH₃CN (174 mg). Après 1 heure 30 minutes d'agitation, on évapore sous pression réduite, reprend le résidu avec du dichlorométhane, lave la phase organique, sèche et amène à sec pour obtenir 1,31 g de produit brut que l'on purifie par chromatographie sur silice en éluant avec le mélange CH₂Cl₂/MeOH/eau 90/10/0,01. On obtient 818 mg de produit attendu. Rf 0,09 CH₂Cl₂/MeOH (85/15).
3,07 (s1, 2H, N-CH₂-CH₂-O) ; 3,81 (m, 2H, N-CH₂-CH₂-O) ; 3,39 (dd) et 3,53 (dd) 2H et 3,83 (m, 2H) : O-CH₂-CH-CH₂-O ; 4,38 (m, 1H, O-CH₂-CH-CH₂-O) ; 4,09 (s1, 2H, Ph-CH₂-N) ; 4,41 et 4,50 (AB, 2H, N-CH₂-Cq) ; 6,79 et 7,41 (AA'BB' 4H, Ph-O) ; 6,92 (s1) et 6,97 (s1, 2H, H4 et H5) ; 7,54 (s1, 1H, H2) ; 7,29 (dd, 1H, Hb) ; 7,47 (d, 1H, Ha) ; 7,62 (d, 1H, Hc) ; 6,24 (s1, 4H, H mobiles).

### Stade b) Cis-2-[[[4-[[2-(2,4-dichlorophényl)-2-(1H-imidazol-1-ylméthyl)-1,3-dioxolan-4-yl]méthoxy]phényl]méthyl](3-phényl-2(E)-propényl)amino]-éthanol

A un mélange constitué de de l'alcool préparé au stade précédent) (300 mg, 0,627 mmol) et de Trans-cinnamaldehyde (111 µl) dans le méthanol (6 ml) on ajoute après 15 minutes, en maintenant un pH de l'ordre de 5,5, le NaBH₃CN (59 mg) et laisse réagir pendant 3 heures 45 minutes sous agitation. On évapore ensuite sous pression réduite et purifie par chromatographie sur silice en éluant avec un mélange CH₂Cl₂/MeOH 95/5 pour obtenir 127 mg de produit attendu. Rf 0,78 CH₂Cl₂/MeOH 90/10.
RMN CDCl₃ 300 MHz
3,23 (dl, 2H, N-CH₂-CH=CH-Ph) ; 6,33 (td, 1H, J=6,5, 16 Hz, N-CH₂-CH=C_{H}-Ph) ; 6,55 (dl, 1H, J=16 Hz, N-CH₂-CH=CH-Ph) 2,53 (masqué, 2H, N-CH₂-CH₂-OH) ; 3,49 (1, 2H, N-CH₂-CH₂-OH) 4,38 (m, 1H, N-CH₂-CH₂-OH) ; 3,58 (m) et 3,69 (m) 2H , 3,68 (m) et 3,88 (dd) 2H : O-CH₂-CH-CH₂-O ; 4, 38 (m, 1H, O-CH₂-CH-CH₂-O) 3,32 (sl, 2H, N-CH₂-Ph) ; 4,51 et 4,56 (AB, 2H, N-CH₂-Cq) ; 7,68 (d, 1H, Ha) ; 7,58 (dd, 1H, Hc) ; 7,46 (m, 1H, Hb) ; 6,81 (sl) et 7,01 (sl) 2H H4 et H5 ; 7,46 (sl, 1H, H2) ; 6,84 et 7,26 (AA'BB' 4H, Ph-O) ; 7,22 (tl, 1H, H en para du phényle) ; 7,32 (tl, 2H, H méta du phényle) ; 7,43 (dl, 2H, H en ortho du phényle).

### De manière similaire aux exemples précédents, on a préparé les produits suivants :

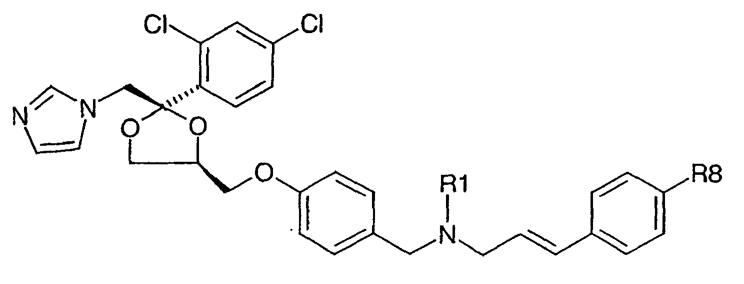

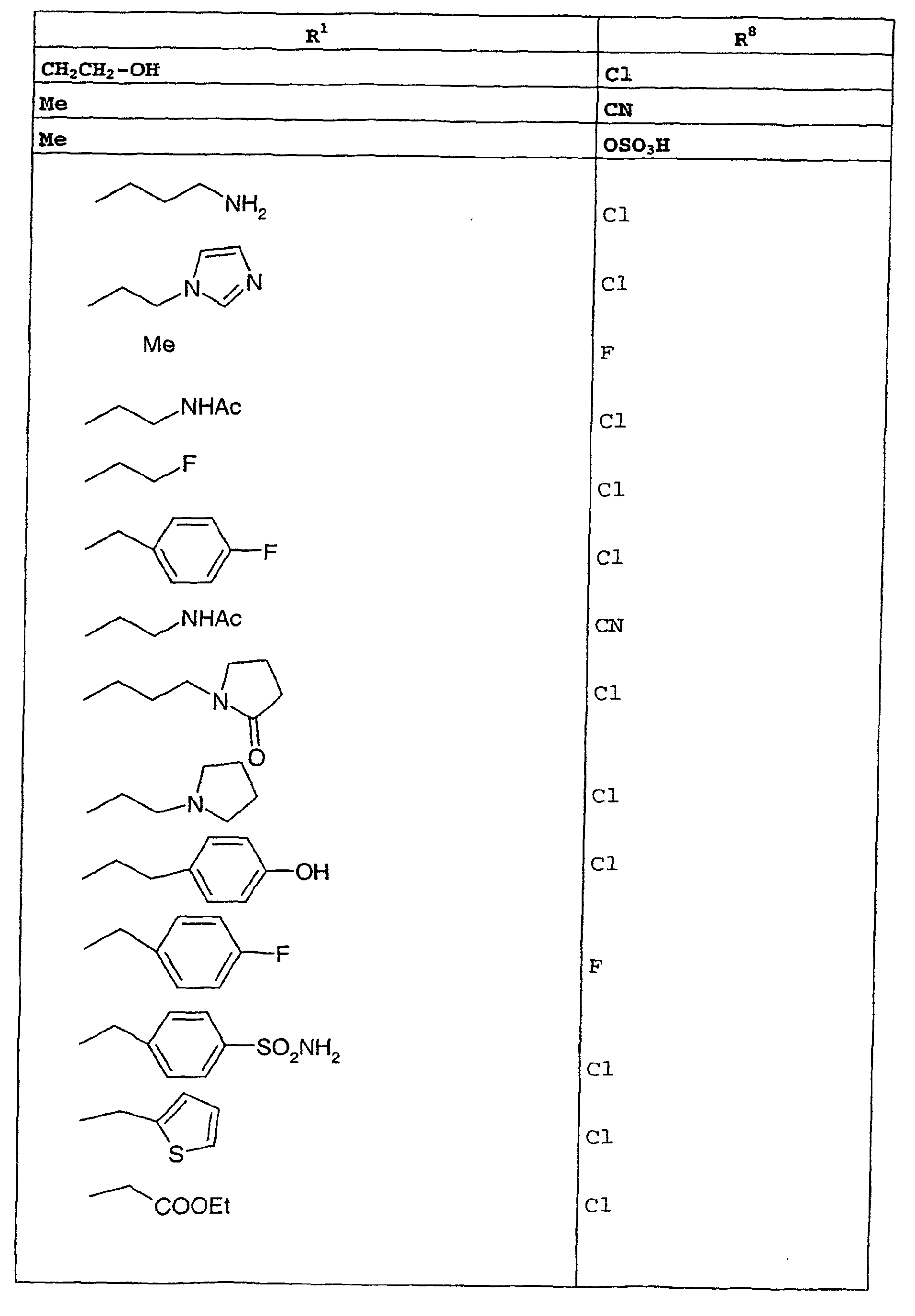

### Compositions pharmaceutiques

On a préparé des composés renfermant

| | |
|---|---|
| Produit de l'exemple 1 | 50 mg |
| Excipient q.s.p. | 1 g |

Détail de l'excipient: amidon, talc, stéarate de magnésium.

### Activité biologique

### 1) Activité antifongique des composés selon l'invention.

On utilise des souris femelles pesant de 18 à 22 g. On leur administre dans la veine de la queue une quantité de *Candida albicans* 44858 à raison de 10⁶CFU par souris (CFU : unité formant des colonies). On sépare les souris en 5 lots de 5 souris et on les traite de la façon suivante :
Une heure après l'infection
   - groupe 1 : les souris sont traitées avec le produit P 25 mg/kg par voie orale
   - groupe 2 : les souris sont traitées avec le produit P par voie intrapéritonéale à raison de 25 mg/kg
   - groupe 3 : les souris sont traitées avec le fluconazole (25 mg/kg par voie orale).
   - groupe 4 : les souris sont traitées avec le fluconazole (25 mg/kg par voie intrapéritonéale).
   - groupe 5 : les souris ne reçoivent aucun traitement antifongique.
Pendant une période de 22 jours, on compte les souris mortes.

### 2) Concentration minimale inhibitrice (CMI)

Des cellules de *Candida albicans* sont préparées comme indiqué dan Journal of Antimicrobial chemotherapy 38, 579-587, lavées 3 fois avec une solution 0,1 M de phosphate et utilisées immédiatement pour déterminer la concentration minimale inhibitrice (CMI).
Les CMI sont déterminés par la modification d'une plaque microtitré selon la méthode standard du Comité National des standards cliniques de laboratoire.
On utilise comme milieu RPMI-1640, et de la L-glutamine tamponnée à pH7 avec une solution 0,15 M de MOPS (acide 3-[N-morpholino]propane sulfonique). On ajoute les cellules de *Candida albicans* (1,5 X 10³ cellules/ml) dans les puits d'une plaque de 96 puits contenant RPMI-1640 et les dilutions d'agents antifongiques. On fait la lecture des résultats 48 heures après incubation à 35°C et on détermine la CMI ou concentration minimale inhibitrice qui inhibe la croissance des cellules du *Candida albicans.*

### Concentration minimale fongicide

Après la lecture à 48 heures des CMI, on secoue les plaques et retire 10 µL d'aliquote des puits que l'on place sur des disques rectangulaires contenant du dextrose agar. Les plaques sont incubées pendant 48 heures à 35°C ; La concentration minimale fongicide et la concentration de l'agent antifongique à laquelle le nombre d'unité formant des colonies est zéro.

### Conclusion

Les composés selon l'invention décrits aux exemples 1 à 10 présentent une activité à < 100 µg/ml dans le test CMI.

## Revendications

1. Les composés de formule (I) 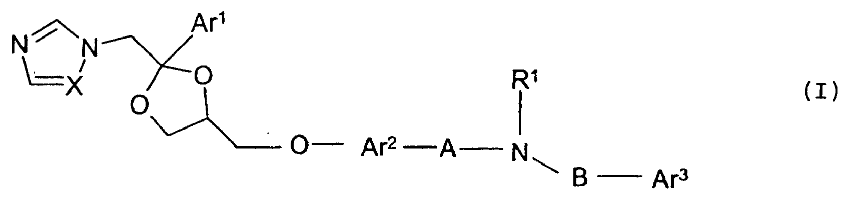
dans laquelle
- X est un atome d'azote ou un groupement CH,
- Ar¹ représente un aryle carbocyclique ou hétérocyclique non substitué ou substitué par un ou plusieurs radicaux R² R³ ou R⁴
- Ar² représente un phénylène ou naphtylène non substitué ou substitué par un ou plusieurs radicaux R⁵, R⁶ ou R⁷
- Ar³ représente un aryle carbocyclique ou hétérocyclique non substitué ou substitué par un ou plusieurs radicaux R⁸, R⁹ ou R¹⁰
- A représente un radical (C₁-C₄)-alkylène ou un radical C(O),
- B représente un radical (C₁-C₄)-alkylène-CH=CH- ou un radical (C₁-C₄)-alkylène-cyclopropylène, les dits radicaux cyclopropylène ou -CH=CH- étant non substitués ou substitués par un radical R² et/ou R³
- R¹ représente un atome d'hydrogène, un groupement -SO₃H ou bien un radical (C₁-C₆)-alkyle non substitué ou substitué par un radical tel que défini pour R²
- R², R³ R⁴, R⁵, R⁶ R⁷, R⁸, R⁹ ou R¹⁰ identiques ou différents, représentent fluor, chlore, brome, cyano, mono- bi- ou trihalogeno (C₁-C₈) alkyle, mono- bi- ou trihalogeno (C₁-C₈)-alkyloxy, hydroxy, nitro, carboxyle, formyle, -SO₃H, -OSO₃H, (R¹¹O)₂ P(O)-, (R¹¹O)₂P(O)-O-, amino, (C₁-C₈)-alkylamino, di((C₁-C₈)alkyl) amino, (C₅-C₁₄)-aryl-(C₁-C₆)-alkylênamino ou (C₅-C₁₄)-arylamino, (C₁-C₈)-alkyle, (C₅-C₁₄)-aryle, un hétérocycle éventuellement substitué par oxo, (C₅-C₁₄)-aryl-(C₁-C₆)alkyle, amino-(C₁-C₆)-alkyle, (C₁-C₈)-alkylamino-(C₁-C₆)-alkyle, di-((C₁-C₈)alkyl) amino-(C₁-C₆)-alkyle, hydroxy- (C₁-C₆) alkyle, (C₁-C₆)-alkyloxy-(C₁-C₆)-alkyle, (C₁-C₈)-alkyloxy éventuellement interrompu par un ou plusieurs atomes d'oxygène, (C₅-C₁₄)-aryl-(C₂-C₆)-alkylènoxy, (C₅-C₂₄)-aryloxy, hydroxy-(C₁-C₆)alkylènoxy, (C₁-C₆)-alkyloxy-(C₁-C₆)alkylènoxy, amino-(C₁-C₆)-alkylènoxy, (C₁-C₆)-alkylamino-(C₁-C₆)-alkylènoxy, di((C₁-C₈)-alkyl) amino-(C₁-C₆)-alkylènoxy, méthylènedioxy, (C₁-C₆)-alkyloxycarbonyle, (C₂-C₆)-alkylcarbonyle, (C₅-C₁₄)aryl-(C₁-C₆)-alkylènecarbonyl, (C₅-C₁₄)-aryl-carbonyle, (C₂-C₆)-alkylaminocarbonyle, (C₂-C₆)alkanoylamino, (C₁-C₆)-alkylsulfonylamino, (C₅-C₁₄)-arylsulfonylamino, (C₅-C₁₄)-aryl-(C₂-C₆)-alkylènesulfonylamino, (C₁-C₆)-alkylaminosulfonyle, (C₅-C₁₄)-aryl-(C₁-C₆)-alkylènaminosulfonyl, (C₁-C₆)-alkylsulfonyl, (C₅-C₁₄)-aryl-(C₁-C₈)-alkylènesulfonyle ou (C₅-C₁₄)-aryl-sulfonyle, les dits radicaux alkyles, aryles ou hétérocycles étant eux même non substitués ou substitués par un ou plusieurs groupements cités ci-dessus.
- R¹¹ représente hydrogène, (C₁-C₁₀)-alkyl, (C₆-C₁₄)-aryle ou (C₆-C₁₄)-aryl-(C₁-C₆)-alkyle,
sous toutes leurs formes stéréoisomères possibles et leurs mélanges,ainsi que leurs sels d'addition physiologiquement acceptables et leurs prodrogues.

2. Les composés de formule (I) tels que définis à la revendication 1, dans lesquels A est un groupement -CH₂-, B est un groupement -CH₂-CH=CH- ou -CH₂-Cyclopropyle- et Ar¹ représente un phényle et Ar² représente un phénylène ainsi que leurs sels d'addition physiologiquement acceptables.

3. Les composés de formule (I) telle que définie à la revendication 1 à la structure : 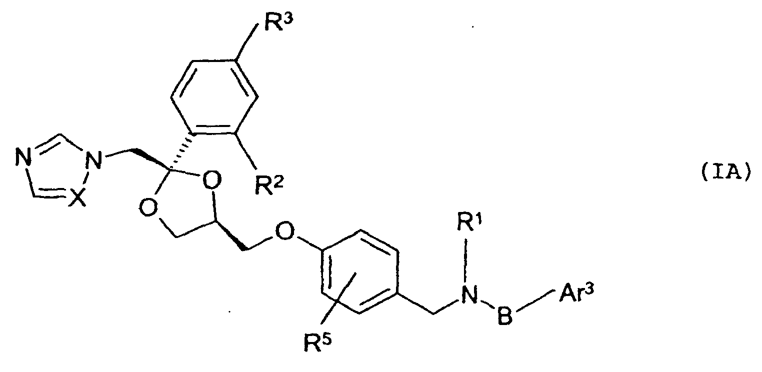
dans laquelle, B, X, Ar³, R⁵ et R¹ sont tels que définis plus haut et R² et R³ représentent un atome d'halogène ainsi que leurs sels d'addition physiologiquement acceptables.

4. Les composés de formule (I) telle que définie à l'une quelconque des revendications 1 à 3 dans lesquels R₂ et R₃ sont des atomes de chlore, X représente CH ou N et Ar³ représente un groupement phényle non substitué ou substitué par R⁸ tel que défini à la revendication 1, ainsi que leurs sels d'addition physiologiquement acceptables.

5. Les composés de formule (I) telle que définie à l'une quelconque des revendications 1 à 4 dans lesquelles, R¹ est un atome d'hydrogène ou un groupement méthyle, ou éthyle non substitué ou substitué par un groupement F, OH, NH₂, (C₁-C₆)-alkyloxy, (C₁-C₆)-alkylamino, di-(C₁-C₆)-alkylamino, pyrrolidino ou 2-oxo-pyrrolidino, ainsi que leurs sels d'addition physiologiquement acceptables.

6. Les composés de formule (I) telle que définie à l'une quelconque des revendications 1 à 5 dans lesquelles Ar³ est un phényle non susbtitué ou substitué par R⁸ représentant un radical -Cl, -F, CN, -CF₃, -OCF₃, -OH, -NH₂, (C₁-C₆)-alkyloxy, (C₁-C₆)-alkylamino, ou di-(C₁-C₆)-alkylamino ou un hétérocycle choisi parmi : 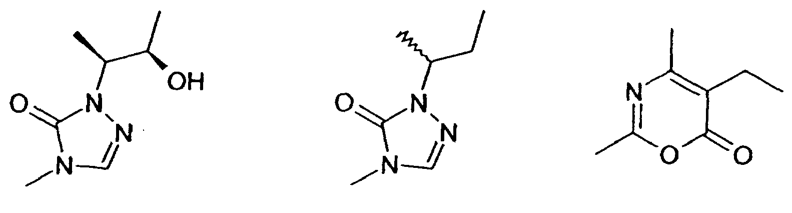 ainsi que leurs sels d'addition physiologiquement acceptables.

7. Les composés de formule (I) telle que définie à la revendication 1 dont les noms suivent :
- cis-4-[[2-(2,4-dichlorophényl)-2-(1H-imidazol-1-ylméthyl)-1,3-dioxolan-4-yl]méthoxy]-N-méthyl-N-(3-phényl-2(E)-propényl)-benzèneméthanamine
- 4-[[2-(2,4-dichlorophényl)-2-(1H-imidazol-1-ylméthyl)-1,3-dioxolan-4-yl]méthoxy]-N-méthyl-N-[3-(4-chloro-phényl-2(E)-propényl]-1-benzeneméthanamine.(?)
- cis-4-[3-[[[4-[[2-(2,4-dichlorophényl)-2-(1H-imidazol-1-ylméthyl)-1,3-dioxan-4-yl]méthoxy]phényl]méthyl]méthylamino]-1(E)-propényl]-phénol
- cis-4-[[2-(2,4-dichlorophényl)-2-(1H-imidazol-1-ylméthyl)-1,3-dioxolan-4-yl]méthoxy]-N-(3-phényl-2(E)-propényl)-benzèneméthanamine
- Phosphate et trifluoroacétate de Cis-4-[3-[[[4-[[2-(2,4-dichlorophényl)-2-(1H-imidazol-1-ylméthyl)-1,3-dioxan-4-yl]méthoxy]phényl]méthyl]méthylamino]-1(E)-propényl]-phénol
- 4-[[2-(2,4-dichlorophényl)-2-(1H-imidazol-1-ylméthyl)-1,3-dioxolan-4-yl]méthoxy]-N-méthyl-N-(3-phényl-2(E)-propényl)-1-naphtalèneméthanamine
- cis-4-[[2-(2,4-dichlorophényl)-2-(1H-imidazol-1-ylméthyl)-1,3-dioxolan-4-yl]méthoxy]-N-(3-phényl-2(E)-propényl)-4-chloro-benzèneméthanamine
- Trifluoroacétate de cis-N-(2-aminoéthyl)-4-[[2-(2,4-dichlorophényl)-2-(1H-imidazol-1-ylméthyl)-1,3-dioxolan-4-yl]méthoxy]-N-(3-phényl-2(E)-propényl)-benzèneméthanamine
- cis-N-(2-aminoéthyl)-N-[3-(4-chlorophényl)-2(E)-propényl]-4-[[2-(2,4-dichlorophényl)-2-(1H-imidazol-1-ylméthyl)-1,3-dioxolan-4-yl]méthoxy]-benzèneméthanamine
- cis-2-[[[4-[[2-(2,4-dichlorophényl)-2-(1H-imidazol-1-ylméthyl)-1,3-dioxolan-4-yl]méthoxy]phényl]méthyl](3-phényl-2(E)-propényl)amino]-éthanol.

8. Un procédé de préparation des composés de formule (I) selon l'une quelconque des revendications 1 à 7 **caractérisé en ce que** l'on soumet un composé de formule (II) : 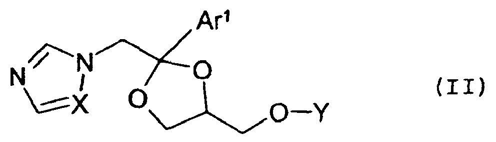
dans laquelle Y représente un groupe partant après substitution nucléophile tel que mésylate ou tosylate et les autres substituants conservent leur signification précédentes à l'action, en présence d'une base, d'un composé de formule (III) :
HO-Ar²-A-N(R¹)-B-Ar³ (III)
dans laquelle Ar², A, R¹, B et Ar³ sont tels que définis à la revendication 1, pour obtenir le composé de formule (I) correspondant.

9. Un procédé de préparation des composés de formule (I) selon l'une quelconque des revendications 1 à 7 **caractérisé en ce que** l'on soumet un composé de formule (II) avec un aryle de formule (III') HO-C₆H₄-CHO, en présence d'une base, le phénylène étant non substitué ou substitué par R⁵ afin d'obtenir un composé de formule (IIa) : 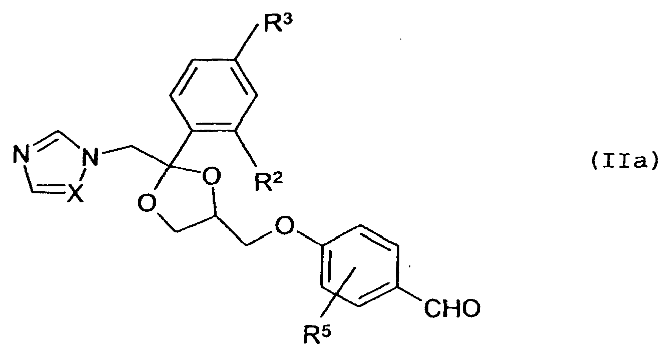 que l'on fait réagir avec une amine R¹-NH₂, R¹ étant tel que défini précédemment, suivi d'une réaction de réduction en présence d'un agent réducteur tel que NaBH₃CN, afin d'obtenir l'amine de formule (IIb) : 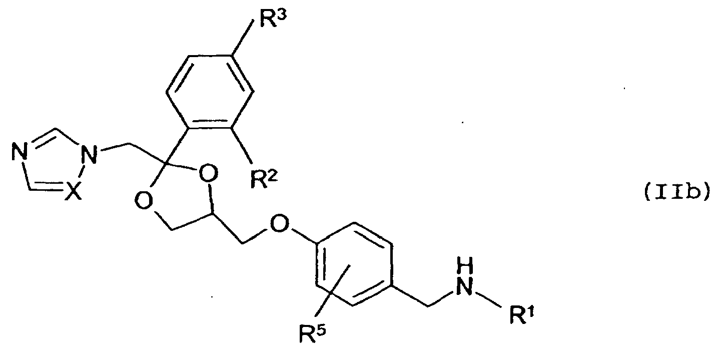 que l'on fait réagir
- soit avec un dérivé de formule :
OHC-CH=CH-C₆H₄-R⁸ ou OHC-(Cyclopropyle)-C₆H₄-R⁸
suivi d'une réaction de réduction en présence d'un agent de réduction tel que NaBH₃CN ou pyridine. BH₃
- soit avec un composé de formule :
AcO-CH₂-CH=CH-C₆H₄-R⁸
en présence d'un dérivé du palladium afin d'obtenir les composés de formules (IAA) et (IAB) suivantes :

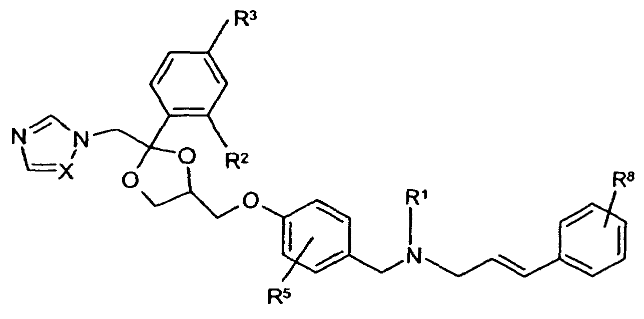

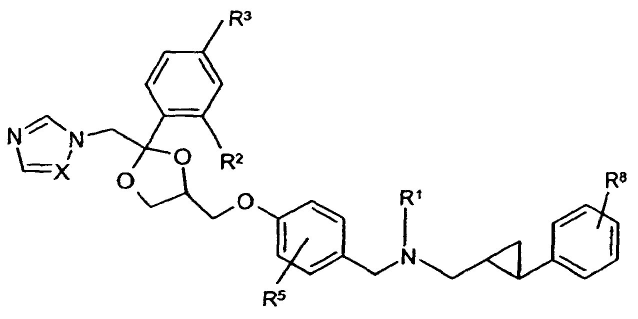

10. Un procédé de préparation des composés de formule (I) selon l'une quelconque des revendications 1 à 7 **caractérisé en ce que** l'on soumet un composé de formule on fait réagir le composé de formule (IIa) tel que défini à la revendication 9 avec une amine de formule R'¹-(CH₂)₂-NH₂, R'¹ représentant un groupement F, OH, une amine ou une alkylamine convenablement protégée, pyrrolidino ou 2-oxo-pyrrolidino ou une dialkylamine afin d'obtenir un composé de formule (IIc) en présence d'un agent réducteur tel que NaBH₃CN 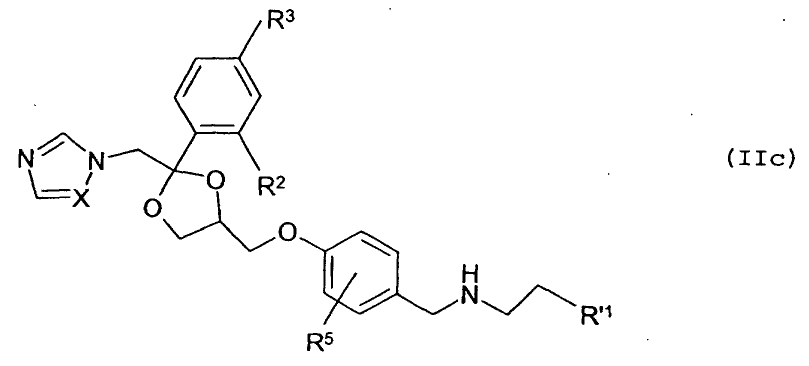 que l'on fait réagir avec une aldéhyde conjuguée telle que définie précédemment afin d'obtenir un composé de formule (IAB) :

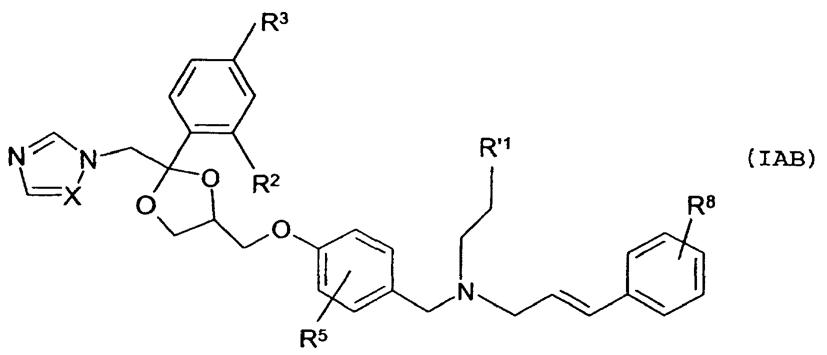

11. A titre de médicament les composés de formule (I) tels que définis à l'une quelconque des revendications 1 à 7 ainsi que leurs sels d'addition pharmaceutiquement acceptables et/ou leurs prodrogues.

12. Les composés de formule (I) tels que définis à l'une quelconque des revendications 1 à 7 ainsi que leurs sels d'addition pharmaceutiquement acceptables et/ou leurs prodrogues pour leur utilisation à titre d'antifongique.

13. Composition pharmaceutique renfermant au moins 1 composé de formule (I) tels que définis à l'une quelconque des revendications 1 à 7 ainsi que leurs sels d'addition pharmaceutiquement acceptables et/ou leurs prodrogues et un véhicule pharmaceutiquement acceptables.

## Patentansprüche

1. Verbindungen der Formel (I) 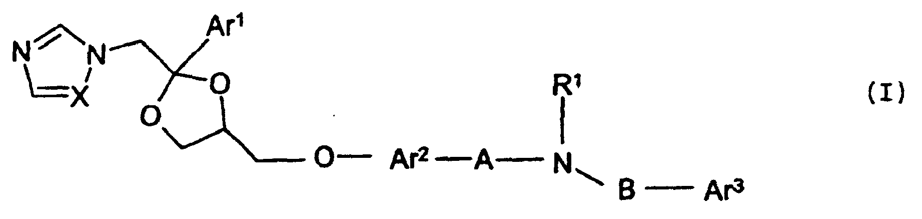 in der
- X ein Stickstoffatom oder eine Gruppe CH ist,
- Ar¹ ein carbocyclisches oder heterocyclisches Aryl darstellt, nicht substituiert oder substituiert durch einen oder mehrere Reste R², R³ oder R⁴,
- Ar² ein Phenylen oder Naphthylen darstellt, nicht substituiert oder substituiert durch einen oder mehrere Reste R⁵, R⁶ oder R⁷,
- Ar³ ein carbocyclisches oder heterocyclisches Aryl darstellt, nicht substituiert oder substituiert durch einen oder mehrere Reste R⁸, R⁹ oder R¹⁰,
- A einen Rest (C₁-C₄)-Alkylen oder einen Rest C(O) darstellt,
- B einen Rest (C₁-C₄)-Alkylen-CH=CH- oder einen Rest (C₁-C₄)-Alkylen-Cyclopropylen darstellt, wobei die genannten Reste Cyclopropylen oder -CH=CH- nicht substituiert oder substituiert sind durch einen Rest R² und/oder R³,
- R¹ ein Wasserstoffatom, eine Gruppe -SO₃H oder auch einen Rest (C₁₋₆)-Alkyl darstellt, nicht substituiert oder substituiert durch einen Rest wie bei R² definiert,
- R², R³, R⁴, R⁵, R⁶, R⁷, R⁸, R⁹ oder R¹⁰, gleich oder verschieden, darstellen: Fluor, Chlor, Brom, Cyano, Mono-, Bi- oder Trihalogeno-(C₁-C₈)-alkyl, Mono-, Bi- oder Trihalogeno-(C₁-C₈)-alkyloxy, Hydroxy, Nitro, Carboxyl, Formyl, -SO₃H, OSO₃H, (R¹¹O)₂P(O)-(R¹¹O)₂P(O) -O-, Amino, (C₁-C₈) -Alkylamino, Di((C₁-C₈)-alkyl)-amino, (C₅-C₁₄)-Aryl-(C₁-C₆)-alkenylamino oder (C₅-C₁₄)-Arylamino, (C₁-C₈)-Alkyl, (C₅-C₁₄)-Aryl, einen Heterocyclus, gegebenenfalls substituiert durch Oxo, (C₅-C₁₄)-Aryl-(C₁-C₆) -alkyl, Amino-(C₁-C₆)-alkyl, (C₁-C₈)-Alkylamino- (C₁-C₆)-alkyl, Di-((C₁-C₈)-alkyl)-amino-(C₁-C₆)-alkyl, Hydroxy-(C₁-C₆)-alkyl, (C₁-C₆)-Alkyloxy-(C₁-C₆)-alkyl, (C₁-C₈)-Alkyloxy, gegebenenfalls unterbrochen durch ein oder mehrere Sauerstoffatome, (C₅-C₁₄)-Aryl-(C₁-C₆)-alkylenoxy, (C₅-C₁₄)-Aryloxy, Hydroxy-(C₁-C₆)-alkylenoxy, (C₁-C₆)-Alkyloxy-(C₁-C₆)-alkylenoxy, Amino-(C₁-C₆)-alkylenoxy, (C₁-C₆)-Alkylamino-(C₁-C₆)-alkylenoxy, Di((C₁-C₆)-alkyl)-amino-(C₁-C₆)-alkylenoxy, Methylendioxy, (C₁-C₆)-Alkyloxycarbonyl, (C₁-C₆)-Alkylcarbonyl, (C₅-C₁₄)-Aryl-(C₁-C₆)-alkylencarbonyl, (C₅-C₁₄)-Arylcarbonyl, (C₁-C₆)-Alkylaminocarbonyl, (C₁-C₆)-Alkanoylamino, (C₁-C₆)-Alkylsulfonylamino, (C₅-C₁₄)-Arylsulfonylamino, (C₅-C₁₄)-Aryl-(C₁-C₆)-alkylensulfonylamino, (C₁-C₆)-Alkylaminosulfonyl, (C₅-C₁₄)-Aryl-(C₁-C₆)-alkylenaminosulfonyl, (C₁-C₆)-Alkylsulfonyl, (C₅-C₁₄)-Aryl-(C₁-C₈)-alkylensulfonyl oder (C₅-C₁₄)-Arylsulfonyl, wobei die genannten Reste Alkyl, Aryl oder die Heterocyclen selbst nicht substituiert oder substituiert sind durch einen oder mehrere der oben genannten Gruppen,
- R¹¹ Wasserstoff, (C₁-C₁₀)-Alkyl, (C₆-C₁₄)-Aryl oder (C₆-C₁₄)-Aryl-(C₁-C₆) -alkyl darstellt,
in allen ihren möglichen stereoisomeren Formen und ihren Mischungen, sowie ihre physiologisch akzeptablen Additionssalze und ihre Prodrugs.

2. Verbindungen der Formel (I) wie in Anspruch 1 definiert, in der A eine Gruppe -CH₂- darstellt, B eine Gruppe -CH₂-CH=CH- oder -CH₂-Cyclopropyl darstellt und Ar¹ ein Phenyl darstellt und Ar² ein Phenylen darstellt, sowie ihre physiologisch akzeptablen Additionssalze.

3. Verbindungen der Formel (I) wie in Anspruch 1 definiert mit der Struktur (IA) 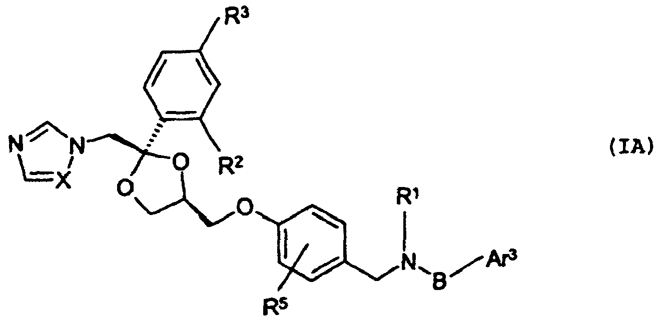 in der
B, X, Ar³, R⁵ und R¹ wie weiter oben definiert sind und R² und R³ Halogenatome darstellen, sowie ihre physiologisch akzeptablen Additionssalze.

4. Verbindungen der Formel (I) wie in irgendeinem der Ansprüche 1 bis 3 definiert, worin R₂ und R₃ Chloratome sind, X CH oder N darstellt und Ar³ eine Gruppe Phenyl darstellt, nicht substituiert oder substituiert durch R⁸ wie bei Anspruch 1 definiert, sowie ihre physiologisch akzeptablen Additionssalze.

5. Verbindungen der Formel (I) wie in irgendeinem der Ansprüche 1 bis 4 definiert, worin R¹ ein Wasserstoffatom oder eine Gruppe Methyl oder Ethyl darstellt, nicht substituiert oder substituiert durch eine Gruppe F, OH, NH₂, (C₁-C₆)-Alkyloxy, (C₁-C₆)-Alkylamino, Di-(C₁-C₆)-alkylamino, Pyrrolidino oder 2-Oxo-pyrrolidino, sowie ihre physiologisch akzeptablen Additionssalze.

6. Verbindungen der Formel (I) wie in irgendeinem der Ansprüche 1 bis 5 definiert, worin Ar³ ein Phenyl ist, nicht substituiert oder substituiert durch R⁸, das einen Rest -Cl, -F, CN, -CF₃, -OCF₃, -OH, -NH₂, (C₁-C₆)-Alkyloxy, (C₁-C₆)-Alkylamino oder Di-(C₁-C₆)-alkylamino oder einen Heterocyclus darstellt, ausgewählt unter: 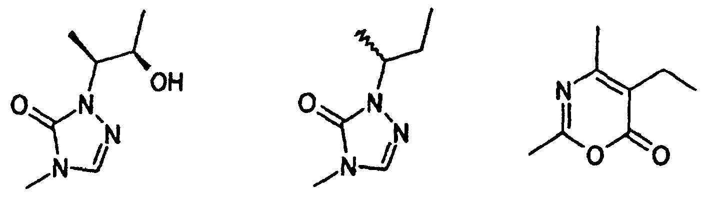 sowie ihre physiologisch akzeptablen Additionssalze.

7. Verbindungen der Formel (I) wie in Anspruch 1 definiert, deren Namen folgen:
- cis-4-{[2-(2,4-Dichlorphenyl)-2-(1H-imidazol-1-ylmethyl)-1,3-dioxolan-4-yl]-methoxy}-N-methyl-N-(3-phenyl-2(E)-propenyl)-benzolmethanamin
- cis-4-{[2-(2,4-Dichlorphenyl)-2-(1H-imidazol-1-ylmethyl)-1,3-dioxolan-4-yl]-methoxy}-N-methyl-N-[3-(4-chlor-phenyl-2(E)-propenyl]-benzolmethanamin (?)
- cis-4-{3-{{[4-[[2-(2,4-Dichlorphenyl)-2-(1H-imidazol-1-ylmethyl)-1,3-dioxan-4-yl]-methoxy]-phenyl]-methyl}-methylamino}-1(E)-propenyl}-phenol
- cis-4-{[2-(2,4-Dichlorphenyl)-2-(1H-imidazol-1-ylmethyl)-1,3-dioxolan-4-yl]-methoxy}-N-(3-phenyl-2(E)-propenyl]-benzolmethanamin
- Phosphat und Trifluoracetat von cis-4-{3-{{[4-[[2-(2,4-Dichlorphenyl)-2-(1H-imidazol-1-ylmethyl)-1,3-dioxan-4-yll-methox-yl-phenyl]-methyl}-methylamino}-1(E)-propenyl}-phenol
- 4-{[2-(2,4-Dichlorphenyl)-2-(1H-imidazol-1-ylmethyl)-1,3-dioxolan-4-yl]-methoxy}-N-methyl-N-(3-phenyl-2(E)-propenyl]-1-naphthalinmethanamin
- cis-4-{[2-(2,4-Dichlorphenyl)-2-(1H-imidazol-1-ylmethyl)-1,3-dioxolan-4-yl]-methoxy}-N-(3-phenyl-2(E)-propenyl]-4-chlor-benzolmethanamin
- Trifluoracetat von cis-N-(2-Aminoethyl)-4-{[2-(2,4-dichlorphenyl)-2-(1H-imidazol-1-ylmethyl)-1,3-dioxolan-4-yl]-methoxy}-N-(3-phenyl-2(E)-propenyl]-benzolmethanamin
- cis-N-(2-Aminoethyl)-N-[3-(4-chlorphenyl)-2(E)-propenyl]-4-{[2-(2,4-dichlorphenyl)-2-(1H-imidazol-1-ylmethyl)-1,3-dioxolan-4-yl]-methoxy}-benzolmethanamin
- cis-2-{{[4-[[2-(2,4-Dichlorphenyl)-2-(1H-imidazol-1-ylmethyl)-1,3-dioxolan-4-yl]-methoxy]-phenyl]-methyl}-(3-phenyl-2(E)-propenyl)-amino}-ethanol.

8. Verfahren zur Herstellung der Verbindungen der Formel (I) nach irgendeinem der Ansprüche 1 bis 7, **dadurch gekennzeichnet, daß** man eine Verbindung der Formel (II) 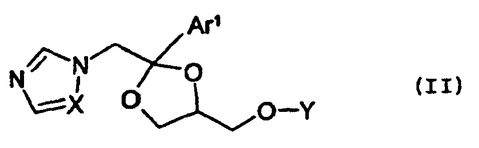 in der Y eine Abgangsgruppe nach nucleophiler Substitution darstellt wie Mesylat oder Tosylat und die anderen Substituenten ihre vorstehenden Bedeutungen beibehalten, der Einwirkung in Anwesenheit einer Base von einer Verbindung der Formel (III)
**HO-Ar**^{**2**}**-A-N (R**^{**1**}**)-B-Ar**^{**3**} **(III)**
unterzieht, in der Ar², A, R¹, B und Ar³ wie in Anspruch 1 definiert sind, um die entsprechende Verbindung der Formel (I) zu erhalten.

9. Verfahren zur Herstellung der Verbindungen der Formel (I) nach irgendeinem der Ansprüche 1 bis 7, **dadurch gekennzeichnet, daß** man eine Verbindung der Formel (II) mit einem Aryl der Formel (III') HO-C₆H₄-CHO in Anwesenheit einer Base zur Reaktion bringt, wobei das Phenylen nicht substituiert oder substituiert ist durch _{R}⁵, um eine Verbindung der Formel (IIa) 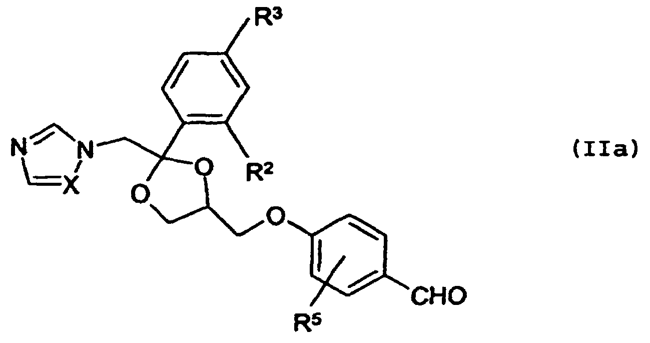 zu erhalten, die man mit einem Amin R¹-NH₂, worin R¹ wie vorstehend definiert ist, zur Reaktion bringt, gefolgt von einer Reduktionsreaktion in Anwesenheit eines Reduktionsmittels wie NaBH₃CN, um das Amin der Formel (IIb) 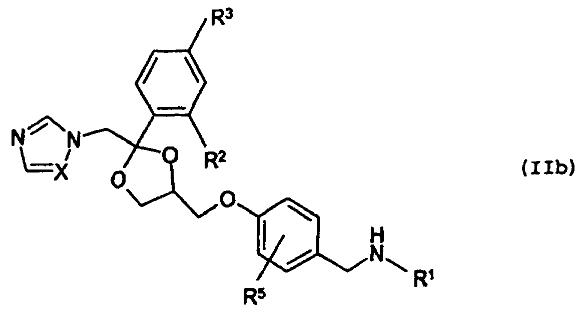 zu erhalten, das man
- entweder mit einem Derivat der Formel
OHC-CH=CH-C₆H₄-R⁸ oder OHC- (Cyclopropyl) -C₆H₄-R⁸
zur Reaktion bringt, gefolgt von einer Reduktionsreaktion in Anwesenheit eines Reduktionsmittels wie NaBH₃CN oder Pyridin.BH₃,
- oder mit einer Verbindung der Formel
AcO-CH₂-CH=CH-C₆H₄-R⁸
in Anwesenheit eines Palladiumderivates zur Reaktion bringt, um die Verbindungen der folgenden Formeln (IAA) und (IAB) 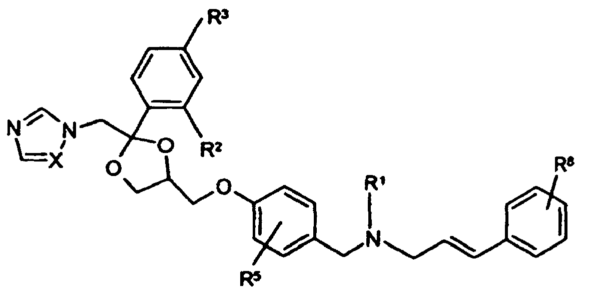 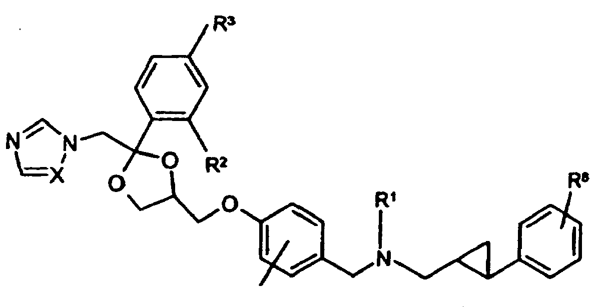 zu erhalten.

10. Verfahren zur Herstellung der Verbindungen der Formel (I) nach irgendeinem der Ansprüche 1 bis 7, **dadurch gekennzeichnet, daß** man eine Verbindung der Formel unterzieht, und zwar die Verbindung der Formel (IIa), wie in Anspruch 9 definiert, mit einem Amin der Formel R'¹-(CH₂)₂-NH₂ zur Reaktion bringt, worin R'¹ eine Gruppe F, OH, ein Amin oder ein Alkylamin, in geeigneter Weise geschützt, Pyrrolidino oder 2-Oxo-pyrrolidino oder ein Dialkylamin darstellt, um eine Verbindung der Formel (IIc) in Anwesenheit eines Reduktionsmittels wie NaBH₃CN 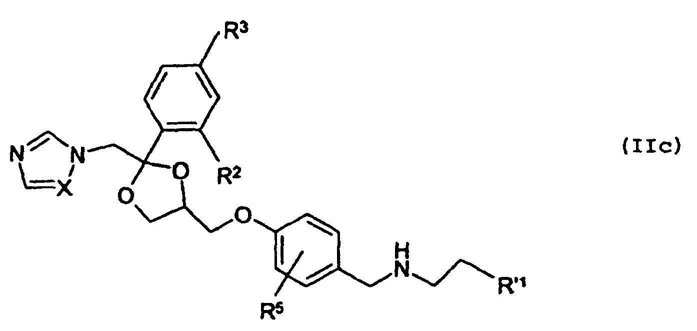 zu erhalten, die man mit einem wie vorstehend definierten konjugierten Aldehyd zur Reaktion bringt, um eine Verbindung der Formel (IAB) 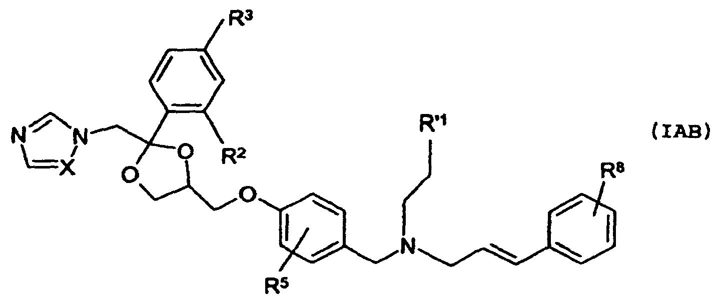 zu erhalten.

11. Als Arzneimittel die Verbindungen der Formel (I) wie in irgendeinem der Ansprüche 1 bis 7 definiert sowie ihre pharmazeutisch akzeptablen Additionssalze und/oder ihre Prodrugs.

12. Verbindungen der Formel (I) wie in irgendeinem der Ansprüche 1 bis 7 definiert sowie ihre pharmazeutisch akzeptablen Additionssalze und/oder ihre Prodrugs zur Verwendung als fungizide Mittel.

13. Pharmazeutische Zusammensetzung, umfassend mindestens eine Verbindung der Formel (I) wie in irgendeinem der Ansprüche 1 bis 7 definiert sowie ihre pharmazeutisch akzeptablen Additionssalze und/oder ihre Prodrugs und einen pharmazeutisch akzeptablen Trägerstoff.

## Claims

1. Compounds of formula (I) 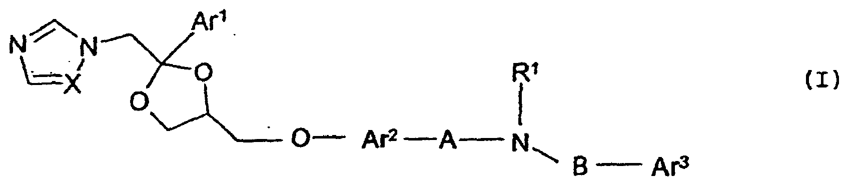
in which
- X is a nitrogen atom or a CH group,
- Ar¹ represents a carbocyclic or heterocyclic aryl that is unsubstituted or substituted with one or more radicals R², R³ or R⁴,
- Ar² represents a phenylene or naphthylene that is unsubstituted or substituted with one or more radicals R⁵, R⁶ or R⁷,
- Ar³ represents a carbocyclic or heterocyclic aryl that is unsubstituted or substituted with one or more radicals R⁸, R⁹ or R¹⁰,
- A represents a (C₁-C₄)alkylene radical or a C(O) radical,
- B represents a (C₁-C₄)alkylene-CH=CH- radical or a (C₁-C₄)alkylene-cyclopropylene radical, said cyclopropylene or -CH=CH- radicals being unsubstituted or substituted with a radical R² and/or R³,
- R¹ represents a hydrogen atom, an -SO₃H group, or a (C₁-C₆)alkyl radical that is unsubstituted or substituted with a radical as defined for R²,
- R², R³, R⁴, R⁵, R⁶, R⁷, R⁸, R⁹ or R¹⁰, which may be identical or different, represent fluorine, chlorine, bromine, cyano, mono-, bi- or trihalo-(C₁-C₈)alkyl, mono-, bi- or trihalo(C₁-C₈)alkyloxy, hydroxyl, nitro, carboxyl, formyl, -SO₃H, -OSO₃H, (R¹¹O)₂P(O)-, (R¹¹O)₂P(O)-O-, amino, (C₁-C₈) alkylamino, di((C₁-C₈)alkyl)amino, (C₅-C₁₄)aryl(C₁-C₆)-alkyleneamino or (C₅-C₁₄)arylamino, (C₁-C₈)alkyl, (C₅-C₁₄)aryl, a heterocycle optionally substituted with oxo, (C₅-C₁₄)aryl (C₁-C₆)alkyl, amino(C₁₋C₆)alkyl, (C₁-C₈)alkylamino (C₁-C₆)alkyl, di((C₁₋C₈)alkyl)amino(C₁-C₆)alkyl, hydroxy(C₁-C₆)alkyl, (C₁-C₆)alkyloxy(C₁-C₆)alkyl, (C₁-C₈)alkyloxy optionally interrupted with one or more oxygen atoms, (C₅-C₁₄)aryl(C₁-C₆)alkyleneoxy, (C₅-C₁₄)aryloxy, hydroxy(C₁-C₆)alkyleneoxy, (C₁-C₆)alkyloxy(C₁₋C₆)alkyleneoxy, amino(C₁-C₆)alkyleneoxy, (C₁-C₆)-alkylamino(C₁-C₆)alkyleneoxy, di((C₁-C₈)alkyl)amino-(C₁-C₆)alkyleneoxy, methylenedioxy, (C₁-C₆) alkyloxycarbonyl, (C₁-C₆)alkylcarbonyl, (C₅-C₁₄)aryl (C₁-C₆)-alkylenecarbonyl, (C₅-C₁₄)arylcarbonyl, (C₁-C₆)-alkylaminocarbonyl, (C₁-C₆)alkanoylamino, (C₁-C₆)-alkylsulphonylamino, (C₅-C₁₄)arylsulphonylamino, (C₅-C₁₄)aryl(C₁-C₆)alkylenesulphonylamino, (C₁-C₆)-alkylaminosulphonyl, (C₅-C₁₄)aryl(C₁-C₆)alkyleneaminosulphonyl, (C₁-C₆)alkylsulphonyl, (C₅-C₁₄)aryl-(C₁-C₈)alkylenesulphonyl or (C₅-C₁₄)arylsulphonyl, said alkyl, aryl or heterocycle radicals being themselves unsubstituted or substituted with one or more groups mentioned above,
- R¹¹ represents hydrogen, (C₁-C₁₀)alkyl, (C₆-C₁₄)aryl or (C₆-C₁₄) aryl (C₁-C₆) alkyl,
in any of their possible stereoisomeric forms and their mixtures, and also their physiologically acceptable addition salts and their prodrugs.

2. Compounds of formula (I) as defined in Claim 1, in which A is a -CH₂- group, B is a -CH₂-CH=CH- or -CH₂₋(cyclopropyl)- group and Ar¹ represents a phenyl and Ar² represents a phenylene, and also their physiologically acceptable addition salts.

3. Compounds of formula (I) as defined in Claim 1, with the structure: 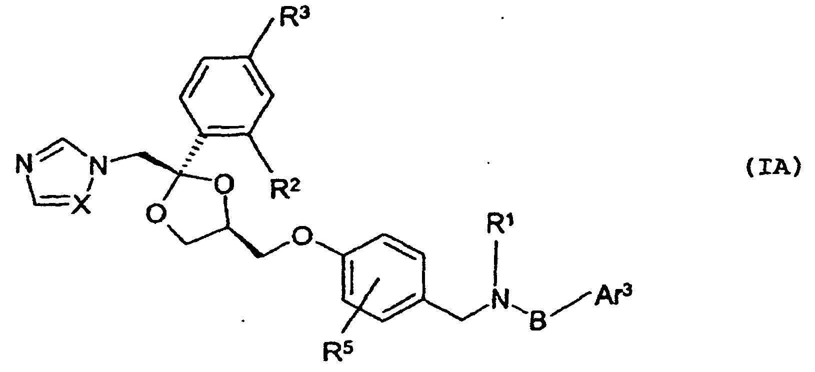
in which B, X, Ar³, R⁵ and R¹ are as defined above and R² and R³ represent a halogen atom, and also their physiologically acceptable addition salts.

4. Compounds of formula (I) as defined in any one of Claims 1 to 3, in which R² and R³ are chlorine atoms, X represents CH or N and Ar³ represents a phenyl group that is unsubstituted or substituted with R⁸ as defined in Claim 1, and also their physiologically acceptable addition salts.

5. Compounds of formula (I) as defined in any one of Claims 1 to 4, in which R¹ is a hydrogen atom or a methyl or ethyl group that is unsubstituted or substituted with an F, OH, NH₂, (C₁-C₆)alkyloxy, (C₁-C₆)-alkylamino, di(C₁-C₆)alkylamino, pyrrolidino or 2-oxopyrrolidino group, and also their physiologically acceptable addition salts.

6. Compounds of formula (I) as defined in any one of Claims 1 to 5, in which Ar³ is a phenyl that is unsubstituted or substituted with R⁸ representing a -Cl, -F, CN, -CF₃, -OCF₃, -OH, -NH₂, (C₁-C₆)alkyloxy, (C₁₋C₆)alkylamino or di(C₁-C₆)alkylamino radical, or a heterocycle chosen from: 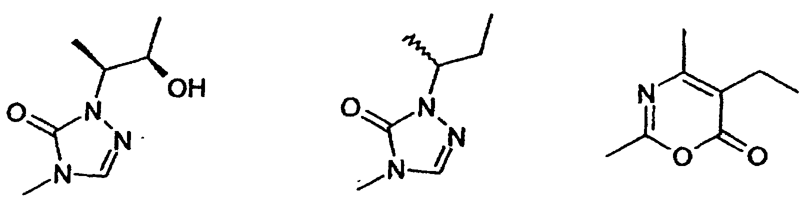 and also their physiologically acceptable salts.

7. Compounds of formula (I) as defined in Claim 1 having the following names:
- cis-4-[[2-(2,4-dichlorophenyl)-2-(1H-imidazol-1-ylmethyl)-1,3-dioxolan-4-yl]methoxy]-N-methyl-N-(3-phenyl-2(E)-propenyl)benzenemethanamine
- 4-[[2-(2,4-dichlorophenyl)-2-(1H-imidazol-1-ylmethyl)-1,3-dioxolan-4-yl]methoxy]-N-methyl-N-[3-(4-chlorophenyl-2(E)-propenyl]-1-benzenemethanamine (?)
- cis-4-[3-[[[4-[[2-(2,4-dichlorophenyl)-2-(1H-imidazol-1-ylmethyl)-1,3-dioxan-4-yl]methoxy]phenyl]-methylamino]-1(E)-propenyl]phenol
- cis-4-[[2-(2,4-dichlorophenyl)-2-(1H-imidazol-1-ylmethyl)-1,3-dioxolan-4-yl]methoxy]-N-(3-phenyl-2(E)-propenyl)benzenemethanamine
- cis-4-[3-[[[4-[[2-(2,4-dichlorophenyl)-2-(1H-imidazol-1-ylmethyl)-1,3-dioxan-4-yl]methoxyphenyl]-methyl]methylamino]-1(E)-propenyl]phenol phosphate trifluoroacetate
- 4-[[2-(2,4-dichlorophenyl)-2-(1H-imidazol-1-ylmethyl)-1,3-dioxolan-4-yl]methoxy]-N-methyl-N-(3-phenyl-2(E)-propenyl)-1-naphthalenemethanamine
- cis-4-[[2-(2,4-dichlorophenyl)-2-(1H-imidazol-1-ylmethyl)1,3-dioxolan-4-yl]methoxy]-N-(3-phenyl-2(E)-propenyl)-4-chlorobenzenemethanamine
- cis-N-(2-aminoethyl)-4-[[2-(2,4-dichlarophenyl)-2-(1H-imidazol-1-ylmethyl)-1,3-dioxolan-4-yl]methoxy]-N-(3-phenyl-2(E)-propenyl)benzenemethanamine trifluoroacetate
- cis-N-(2-aminoethyl)-N-[3-(4-chlorophenyl)-2(E)-propenyl]-4[[2-(2,4-dichlorophenyl)-2-(1H-imidazol-1-ylmethyl)-1,3-dioxolan-4-ylmethoxy]benzenemethanamine
- cis-2-[[[4-[[2-(2,4-dichlorophenyl)-2-(1H-imidazol-1-ylmethyl)-1,3-dioxolan-4-yl]methoxy]phenyl]methyl](3-phenyl-2(E)-propenyl)amino]ethanol.

8. Method for preparing the compounds of formula (I) according to any one of Claims 1 to 7, **characterized in that** a compound of formula (II) 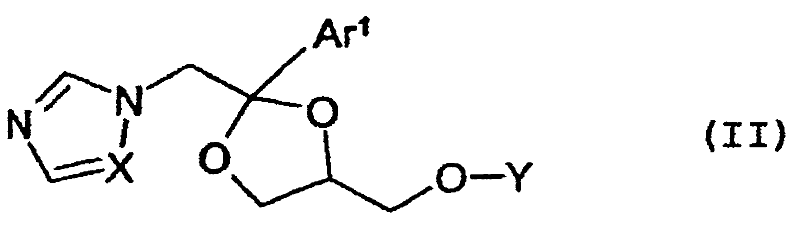
in which Y represents a leaving group after nucleophilic substitution, such as mesylate or tosylate, and the other substituents conserve their meaning above, is subjected to the action, in the presence of a base, of a compound of formula (III):
HO-Ar²-A-N(R¹)-B-AR³ (III)
in which Ar², A, R¹, B and Ar³ are as defined in Claim 1, so as to obtain the corresponding compound of formula (I).

9. Method for preparing the compounds of formula (I) according to any one of Claims 1 to 7, **characterized in that** a compound of formula (II) is subjected to an aryl of formula (III') HO-C₆H₄-CHO, in the presence of a base, the phenylene being unsubstituted or substituted with R⁵, in order to obtain a compound of formula (IIa) 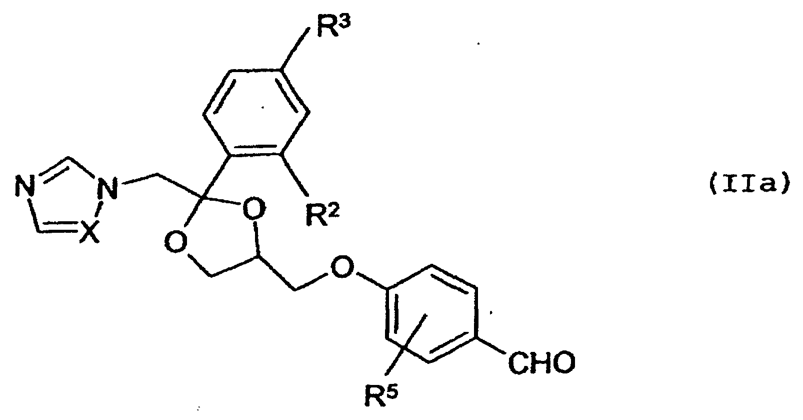 which is reacted with an amine R¹-NH₂, R¹ being as defined above, followed by a reduction reaction in the presence of a reducing agent such as NaBH₃CN, in order to obtain the amine of formula (IIb) 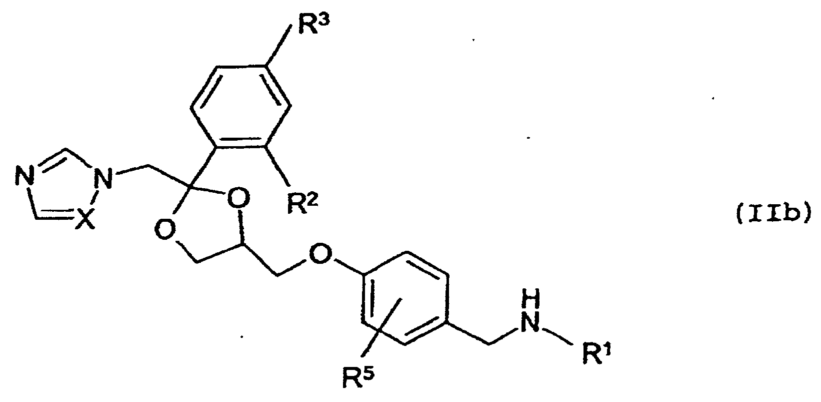 which is reacted
- either with a derivative of formula:
OHC-CH=CH-C₆H₄-R⁸ or OHC-(cyclopropyl)-C₆H₄-R⁸
followed by a reduction reaction in the presence of a reducing agent such as NaBH₃CN or BH₃.pyridine
- or with a compound of formula:
AcO-CH₂-CH=CH-C₆H₄-R⁸
in the presence of a palladium derivative in order to obtain the compounds of formulae (IAA) and (IAB) below:

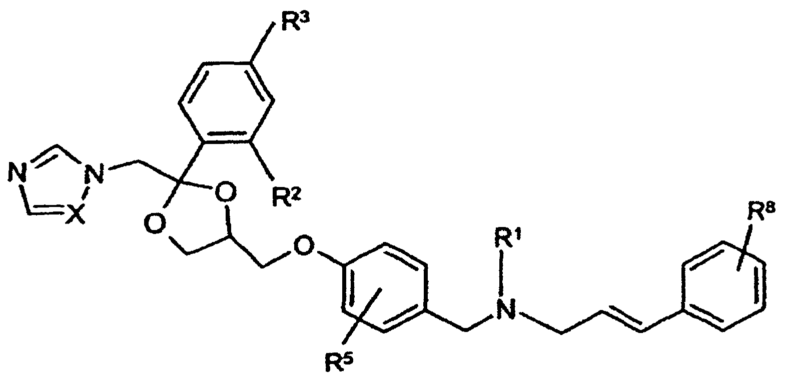

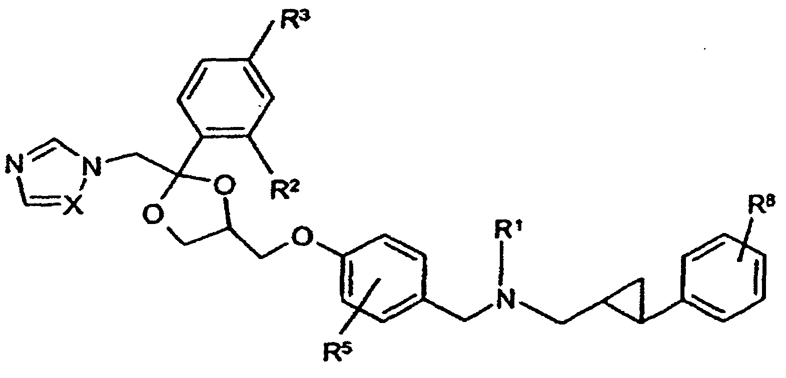

10. Method for preparing the compounds of formula (I) according to any one of Claims 1 to 7, **characterized in that** a compound of formula is subjected the compound of formula (IIa) as defined in Claim 9 is reacted with an amine of formula R'¹-(CH₂)₂-NH₂, R'¹ representing an F or OH group, an amine or an alkylamine that is suitably protected, pyrrolidino or 2-oxopyrrolidino or a dialkylamine, in order to obtain a compound of formula (IIc) in the presence of a reducing agent such as NaBH₃CN 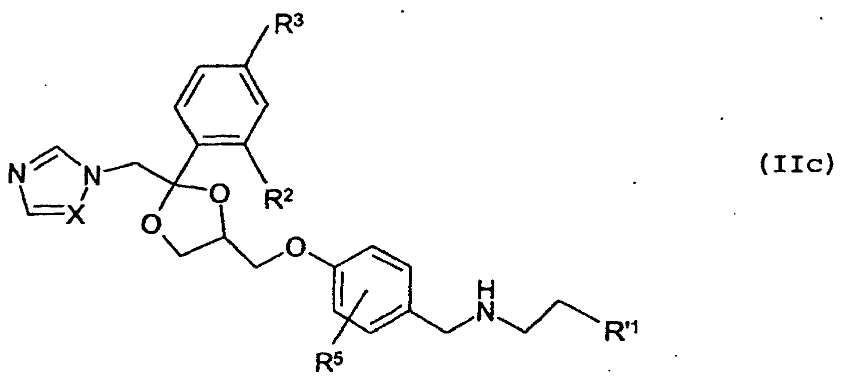 which is reacted with a conjugated aldehyde as defined above, in order to obtain a compound of formula (IAB):

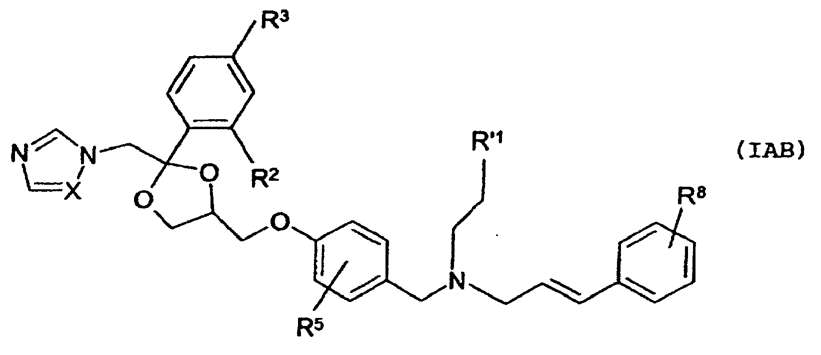

11. Compounds of formula (I) as defined in any one of Claims 1 to 7, and also their pharmaceutically acceptable addition salts and/or their prodrugs, as a medicinal product.

12. Compounds of formula (I) as defined in any one of Claims 1 to 7, and also their pharmaceutically acceptable addition salts and/or their prodrugs, for their use as an antifungal agent.

13. Pharmaceutical composition containing at least one compound of formula (I) as defined in any one of Claims 1 to 7, and also their pharmaceutically acceptable addition salts and/or their prodrugs, and a pharmaceutically acceptable vehicle.
